# EUROPÄISCHE PATENTSCHRIFT

(11) **EP 0 688 784 B1**
(45) Veröffentlichungstag und Bekanntmachung des Hinweises auf die Patenterteilung: **14.12.2005**
(21) Anmeldenummer: 95108896.2
(22) Anmeldetag: 09.06.1995
(51) Int. Cl.: C07H 21/00, A61K 31/70, C12Q 1/68

(54) **3'-Modifizierte Oligonucleotid-Derivate**
3'-Modified oligonucleotide derivatives
Dérivés d'oligonucléotides modifiés en position 3'

(30) Priorität: 15.06.1994 DE 4420737; 09.07.1994 DE 4424263
(43) Veröffentlichungstag der Anmeldung: 27.12.1995
(73) Patentinhaber: Sanofi-Aventis Deutschland GmbH, 65929 Frankfurt am Main (DE)
(72) Erfinder: Peyman, Anuschirwan, Dr., D-65779 Kelkheim (DE); Uhlmann, Eugen, Dr., D-61479 Glashütten (DE); Carolus, Carolin, D-65321 Frankfurt (DE)

(56) Entgegenhaltungen:
- NUCLEIC ACIDS RESEARCH, Bd. 18, Nr. 8, 25.April 1990, OXFORD GB, Seiten 2065-2068, XP002018065 W.WILK ET AL.: "Backbone Modified Oligonucleotides Containing a Butanediol-1,3 Moiety as a "Vicarious Segment" for the Deoxyribosyl Moiety - Synthesis and Enzyme Studies."
- NUCLEIC ACIDS RESEARCH, Bd. 15, Nr. 7, 10.April 1987, OXFORD GB, Seiten 3113-3129, XP002018066 F.SEELA ET AL.: "Oligodeoxyribonucleotides Containing 1,3-Propanediol as Nucleoside Substitute."
- PROCEEDINGS OF THE NATIONAL ACADEMY OF SCIENCES OF USA, Bd. 90, Nr. 4, 15.Februar 1993, WASHINGTON US, Seiten 1300-1304, XP002018067 P.F.TORRENCE ET AL.: "Targeting RNA for Degrasation with a (2',5')Oligoadenylate-Antisense Chimera."
- PROCEEDINGS OF THE NATIONAL ACADEMY OF SCIENCES OF USA, Bd. 86, Nr. 10, Mai 1989, WASHINGTON US, Seiten 3614-3618, XP002018068 G.VESNAVER ET AL.: "Influence of Abasic and Anucleosidic Sites on the Stability, Conformation, and Melting Behaviour of a DNA Duplex : Correlations of Thermodynamic and Structural Data."
- JOURNAL OF THE AMERICAN CHEMICAL SOCIETY, Bd. 113, Nr. 13, 19.Juni 1991, DC US, Seiten 5109-5111, XP002018069 P.L.RICHARDSON ET AL.: "Tethered Oligonucleotide Probes. Strategy for the Recognition of Structured RNA."
- NUCLEOSIDES AND NUCLEOTIDES, Bd. 13, Nr. 10, 1994, Seiten 2245-2265, XP002018070 M.J.DE VOS ET AL.: "New Non Nucleosidic Phosphoramidites for the Solid Phase Multi-Labelling of Oligonucleotides : Comb- and Multifork-Like Structures."
- MISIURA K. ET AL: 'Biotinyl and phosphotyrosinyl phosphoramidite derivatives useful in the incorporation of multiple reporter groups on synthetic oligonucleotides' NUCLEIC ACID RESEARCH Bd. 18, Nr. 15, 1990, Seiten 4345 - 4354

## Beschreibung

Die vorliegende Erfindung betrifft neue Oligonucleotidanaloga mit wertvollen physikalischen, biologischen und pharmakologischen Eigenschaften. Ihre Anwendung bezieht sich auf die Verwendung als Inhibitoren der Genexpression (Antisense Oligonucleotide, Ribozyme, Sense Oligonucleotide und Triplex Forming Oligonucleotide), als Sonden zum Nachweis von Nucleinsäuren und als Hilfsmittel in der Molekularbiologie.

Oligonucleotide finden in wachsendem Maße Anwendung als Inhibitoren der Genexpression (J. F. Milligan, M. D. Matteucci und J. C. Martin, J. Med. Chem. 36 (1993) 1923; E. Uhlmann and A. Peyman, Chemical Reviews 90 (1990) 543).

Antisense Oligonucleotide sind Nucleinsäure-Fragmente, deren Basensequenz komplementär ist zu einer zu inhibierenden mRNA. Diese Target-mRNA kann zellulären, viralen oder sonstigen pathogenen Ursprungs sein. Als zelluläre Target-Sequenzen kommen beispielsweise die von Rezeptoren, Enzymen, Wachstumsfaktoren, Immunmodulatoren, Ionenkanälen oder Onkogenen in Frage. Die Inhibition der Virus Vermehrung mit Hilfe von Antisense Oligonucleotiden wurde beispielsweise für RSV (Rous Sarcoma Virus), HSV-1 und -2 (Herpes Simplex Virus Typ I und II), HIV (Human Immunodeficiency Virus) und Influenza-Viren beschrieben. Dabei setzt man Oligonucleotide ein, die zur viralen Nucleinsäure komplementär sind.

Sense Oligonucleotide sind dagegen in ihrer Sequenz so konzipiert, daß sie beispielsweise Nucleinsäure-bindende Proteine oder Nucleinsäure-prozessierende Enzyme binden ("einfangen") und so deren biologische Aktivität inhibieren (C. Hélène and J. J. Toulmé, Biochim. Biophys. Acta 1049 (1990) 99). Als virale Targets sind hier beispielsweise die Reverse Transkriptase, DNA-Polymerase und Transaktivator-Proteine zu nennen. Triplex Forming Oligonucleotide haben im allgemeinen die DNA als Target und bilden nach Bindung an diese eine tripelhelicale Struktur aus.

Während mit Hilfe der Antisense Oligonucleotide im allgemeinen die Prozessierung (Splicing etc.) der mRNA oder deren Translation in das Protein gehemmt werden, hemmen Triplex Forming Oligonucleotide die Transkription oder Replikation der DNA (N. T. Thuong und C. Hélène, Angew. Chem. 105 (1993) 697; Uhlmann und Peyman, Chemical Reviews 90 (1990) 543). Es ist aber auch möglich, einzelsträngige Nucleinsäuren in einer ersten Hybridisierung mit einem Antisense Oligonucleotid unter Ausbildung eines Doppelstranges zu binden, der dann in einer zweiten Hybridisierung mit einem Triplex Forming Oligonucleotid eine Triplex-Struktur ausbildet. Die Antisense und Triplex Bindungsregionen können dabei entweder in zwei separaten Oligonucleotiden oder aber in einem Oligonucleotid beherbergt sein.

Eine weitere Anwendung synthetischer Oligonucleotide sind die sogenannten Ribozyme, welche die Target-RNA infolge ihrer Ribonuclease-Aktivität zerstören (D. Castanotto, J. J. Rossi, J. O. Deshler, Critical Rev. Eukar. Gene Expr. 2 (1992) 331).

In der DNA-Diagnostik werden Nucleinsäure-Fragmente mit geeigneter Markierung als sogenannte DNA-Sonden oder DNA-Probes für die spezifische Hybridisierung an eine nachzuweisende Nucleinsäure eingesetzt. Die spezifische Ausbildung des neuen Doppelstranges wird dabei mit Hilfe der Markierung, die vorzugsweise nicht radioaktiv ist, verfolgt. Auf diese Weise lassen sich genetische, maligne, virale oder durch andere Pathogene verursachte Krankheiten nachweisen.

Für die meisten genannten Anwendungen sind Oligonucleotide in ihrer natürlich vorkommenden Form wenig geeignet oder völlig ungeeignet. Sie müssen chemisch so modifiziert werden, daß sie den speziellen Anforderungen gerecht werden. Damit Oligonucleotide in biologischen Systemen, beispielsweise zur Inhibition der Virus-Vermehrung eingesetzt werden können, müssen sie folgende Voraussetzungen erfüllen:
1. Sie müssen unter in vivo Bedingungen, also sowohl im Serum als auch intrazellulär, eine ausreichend große Stabilität aufweisen.
2. Sie müssen so beschaffen sein, das sie die Zell- und Nucleus-Membran passieren können.
3. Sie müssen unter physiologischen Bedingungen in Basen- spezifischer Weise an ihre Target-Nucleinsäure binden, um den inhibitorischen Effekt zu entfalten.

Für DNA-Sonden sind diese Voraussetzungen nicht unabdingbar; jedoch müssen diese Oligonucleotide so derivatisiert sein, daß ein Nachweis, beispielsweise mittels Fluoreszenz, Chemilumineszenz, Kolorimetrie oder spezifischer Färbung, möglich ist (Beck und Köster, Anal. Chem. 62 (1990) 2258).

Die chemische Veränderung der Oligonucleotide erfolgt meistens in der Weise, daß Phosphatrückgrat, Ribose-Einheit oder die Nucleobasen entsprechend verändert werden (Uhlmann und Peyman, Chemical Reviews 90 (1990) 543). Eine weitere häufig genutzte Methode ist die Herstellung von Oligonucleotid-5'-Konjugaten durch Umsetzung der 5'-Hydroxy-Gruppe mit entsprechenden Phosphorylierungs-Reagenzien. Oligonucleotide, die nur am 5'-Ende modifiziert sind, haben den Nachteil, daß sie im Serum abgebaut werden. Wenn dagegen alle Internucleotid-Phosphat-Reste verändert werden, verändern sich die Eigenschaften der Oligonucleotide oft drastisch. Beispielsweise ist die Löslichkeit der Methylphosphonat Oligonucleotide in wäßrigem Medium vermindert und das Hybridisierungsvermögen reduziert. Phosphorothioat-Oligonucleotide wirken unspezifisch, so daß beispielsweise auch Homooligomere (Uhlmann und Peyman, Chemical Reviews 90 (1990) 543) K MISIURA ET AL., (NUCLEIC ACID RESEARCH, , 1990, Band 18, Nr. 15, Seitein 4345 - 4354) offenbart (S. 4346, Spalte 1, Z. 6-16; S. 4347, Spalte 1, Z. 6-10; S. 4348, Spalte 2, Z. 1-2) Oligonukleotide welche an internen Positionen und/oder am 5'-Ende mit DNA Markierungssonden wie Biotinyl-Linker Einheiten und Phosphotyrosinyl-Linker-Einheiten gemodifiziert sind und welche mit Hilfe konventioneller Festphasensynthese ausgehend von Phosphoramidit-Bausteinen hergestellt werden.

Der Abbau von Oligonucleotiden durch 3'-nucleolytische Aktivität wird allgemein als der vorwiegende Abbau durch Nucleasen im Serum angesehen. Aufgabe ist es daher, 3'-derivatisierte Oligonucleotidanaloga mit spezifischer Wirksamkeit, erhöhter Serumstabilität und guter Löslichkeit bereitzustellen.

Gegenstand dieser Erfindung sind daher Verbindungen der Formel I 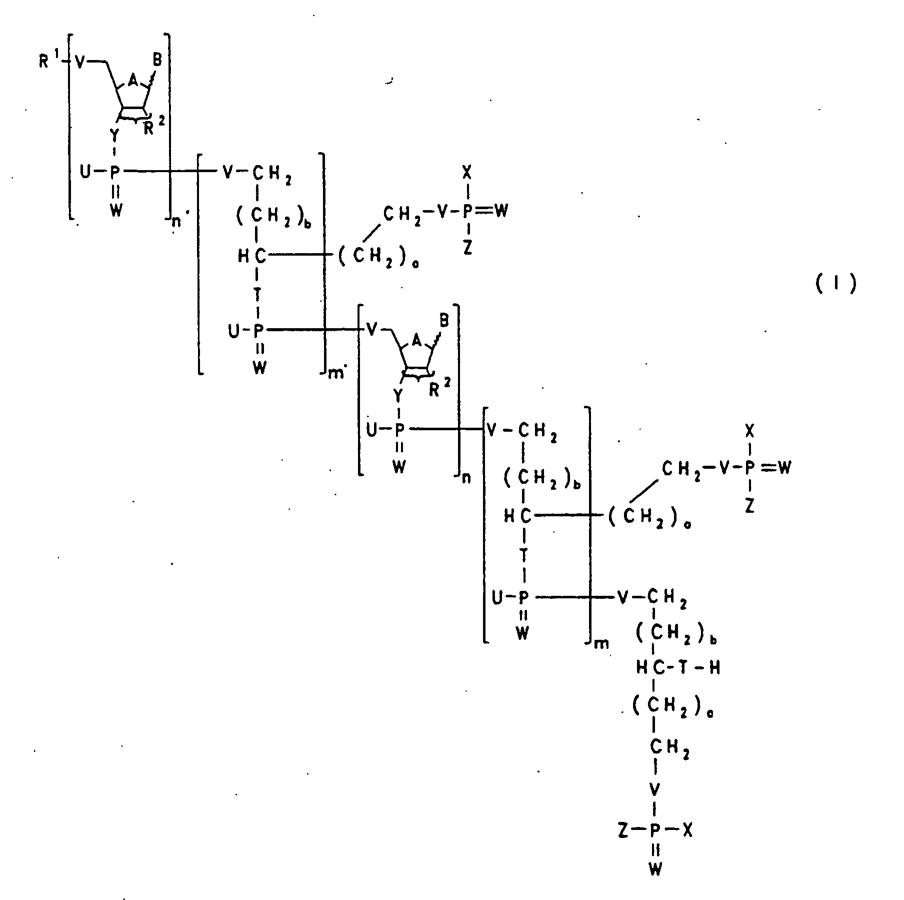 sowie deren physiologisch verträglichen Salze, worin
- a: eine Zahl von Null bis 20, bevorzugt von Null bis 10, besonders bevorzugt von Null bis 6, ganz besonders bevorzugt von Null bis 4 bedeutet;
- b: eine Zahl von Null bis 20, bevorzugt von Null bis 10, besonders bevorzugt von Null bis 4, ganz besonders bevorzugt von Null bedeutet;
- R¹: Wasserstoff, C₁-C₁₈-Alkyl, vorzugsweise C₁-C₆-Alkyl, insbesondere Methyl, C₂-C₁₈-Alkenyl, C₃-C₁₈-Alkinyl, C₁-C₁₈-Alkylcarbonyl, C₂-C₁₉-Alkenylcarbonyl, C₃-C₁₉-Alkinylcarbonyl, C₆-C₂₀-Aryl, C₆-C₁₄-Aryl-C₁-C₈-alkyl, oder einen Rest der Formel III 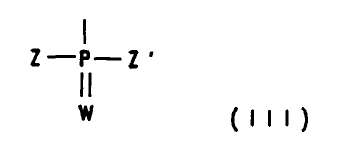 bedeutet, bevorzugt Wasserstoff oder einen Rest der Formel III, ganz besonders bevorzugt Wasserstoff;
- R²: Wasserstoff, Hydroxy, C₁-C₁₈-Alkoxy, Halogen, Azido oder NH₂, bevorzugt Wasserstoff, Hydroxy, C₁-C₄-Alkoxy, Fluor oder NH₂, besonders bevorzugt Wasserstoff oder Hydroxy, ganz besonders bevorzugt Wasserstoff bedeutet;
- B: für eine in der Nucleotidchemie übliche Base, beispielsweise für natürliche Basen wie Adenin, Cytosin, Guanin, Uracil und Thymin oder unnatürliche Basen wie beispielsweise Purin, 2.6-Diaminopurin, 7-Deazaadenin, 7-Deazaguanin, N⁴N⁴-Ethanocytosin, N⁶N⁶-Ethano-2.6-diaminopurin, Pseudoisocytosin, 5-Propinuracil, 5-Propincytosin, 5-Fluorocytosin, 5-Fluoruracil, 5-Hydroxymethyluracil und 5-Bromcytosin bzw. ganz besonders bevorzugt für Adenin, Cytosin, Guanin, Uracil, Thymin, 5-Propinuracil und 5-Propincytosin steht;
- n: eine ganze Zahl von 7 bis 25 bedeutet;
- n': eine ganze Zahl von Null bis 50, bevorzugt Null bis 40, besonders bevorzugt Null bis 30, ganz besonders bevorzugt Null bis 25 bedeutet;
- m: eine ganze Zahl von Null bis 5, ganz besonders bevorzugt Null bedeutet;
- m': in Formel I eine ganze Zahl von Null bis 5, ganz besonders bevorzugt Null oder 1 bedeutet;
- A: für Oxy, Thioxy oder Methylen, bevorzugt für Oxy steht;
- W: Oxo, Thioxo oder Selenoxo, bevorzugt Oxo oder Thioxo, besonders bevorzugt Oxo bedeutet;
- V: Oxy oder Sulfandiyl, ganz besonders bevorzugt Oxy bedeutet;
- T: Oxy, Sulfandiyl oder Imino, ganz besonders bevorzugt Oxy bedeutet;
- Y: Oxy, Sulfandiyl, Imino oder Methylen, ganz besonders bevorzugt Oxy bedeutet;
- X: Hydroxy oder Mercapto bedeutet;
- U: Hydroxy, Mercapto, BH₃, SeH, C₁-C₁₈-Alkoxy, vorzugsweise C₁-C₆-Alkoxy, C₁-C₁₈-Alkyl, vorzugsweise C₁-C₆-Alkyl, C₆-C₂₀-Aryl, C₆-C₁₄-Aryl-C₁-C₈-alkyl, NHR³, NR³R⁴ oder einen Rest der Formel IV

(OCH₂CH₂)ₚO(CH₂)_{q}CH₂R⁵ (IV)

bevorzugt Hydroxy, Mercapto, C₁-C₆-Alkoxy, C₁-C₆-Alkyl, NR³R⁴ oder NHR³ und besonders bevorzugt Hydroxy oder C₁-C₆-Alkyl bedeutet, worin
- R³: C₁-C₁₈-Alkyl, vorzugsweise C₁-C₈-Alkyl, C₆-C₂₀-Aryl, C₆-C₁₄-Aryl-C₁-C₈-alkyl, -(CH₂)_{c}-[NH(CH₂)_{c}]_{d}-NR⁶R⁶, worin c eine ganze Zahl von 2 bis 6 und d eine ganze Zahl von Null bis 6 ist, und R⁶ unabhängig voneinander Wasserstoff, C,-C₆-Alkyl oder C₁-C₄-Alkoxy-C₁-C₆-alkyl, vorzugsweise Methoxyethyl, bevorzugt C₁-C₈-Alkyl, insbesondere C₁-C₄-Alkyl bedeutet;
- R⁴: C₁₋C₁₈-Alkyl, C₆-C₂₀-Aryl oder C₆-C₁₀-Aryl-C₁-C₈-alkyl, bevorzugt C₁-C₈-Alkyl, insbesondere C₁-C₄-Alkyl, C₆-C₂₀-Aryl oder C₆-C₁₀-Aryl-C₁-C₈-alkyl, bedeutet
oder im Falle von NR³R⁴ zusammen mit R³ und dem sie tragenden Stickstoffatom einen 5-6-gliedrigen heterozyklischen Ring bedeutet, der zusätzlich ein weiteres Heteroatom aus der Reihe O, S, N enthalten kann;
- p: eine ganze Zahl von 1 bis 100, bevorzugt 3 bis 20 und besonders bevorzugt 3 bis 8, ist;
- q: eine ganze Zahl von Null bis 22, bevorzugt Null bis 15, ist;
- R⁵: Wasserstoff oder eine funktionelle Gruppe ausgewählt aus Hydroxy, Amino, NHR⁷, COOH, CONH₂, COOR⁸ oder Halogen bedeutet, worin R⁷ C₁-C₆-Alkyl und R⁸ C₁-C₄-Alkyl, vorzugsweise Methyl, bedeutet;
- Z, Z': unabhängig voneinander Hydroxy, Mercapto, SeH, C₁-C₂₂-Alkoxy, vorzugsweise C₆-C₁₈-Alkoxy, -O-(CH₂)_{b}-NR⁷R⁸, worin b eine ganze Zahl von 1 bis 6 ist, und R⁷ C₁-C₆-Alkyl und R⁸ C₁-C₄-Alkyl ist oder R⁷ und R⁸ zusammen mit dem sie tragenden Stickstoffatom einen 3-6 gliedrigen Ring bilden; C₁-C₁₈-Alkyl, vorzugsweise C₁-C₈-Alkyl, C₆-C₂₀-Aryl, C₆-C₁₄-Aryl-C₁-C₈-alkyl, vorzugsweise C₆-C₁₀-Aryl-C₁-C₄-alkyl, C₆-C₁₄-Aryl-C₁-C₈-alkoxy, vorzugsweise C₆-C₁₀-Aryl-C₁-C₄-alkoxy, wobei Aryl auch Heteroaryl bedeutet und Aryl gegebenenfalls durch 1, 2 oder 3 gleiche oder verschiedene Reste aus der Reihe Carboxy, Amino, Nitro, C₁-C₄-Alkylamino, C₁-C₆-Alkoxy, Hydroxy, Halogen und Cyano substituiert ist, C₁-C₁₈-Alkylmercapto, NHR³, NR³R⁴, worin R³ und R⁴ wie oben definiert sind, oder eine untergenannte Gruppe bedeuten, die die intrazelluläre Aufnahme begünstigt oder als Markierung einer DNA-Sonde dient oder bei der Hybridisierung des Oligonucleotidanalogons an die Target-Nucleinsäure diese unter Bindung, Quervernetzung oder Spaltung angreift, oder ein über das 5'- oder 3'-Ende verknüpftes Nucleosid oder Oligonucleotid; und
die geschweifte Klammer andeutet, daß sich R² und der benachbarte Phosphorylrest in 2'- und 3'-Stellung oder auch umgekehrt in 3'- und 2'-Stellung befinden können,
wobei jedes Nucleotid in seiner D- bzw. L-Konfiguration vorliegen kann und sich die Base B in α- bzw. β-Stellung befinden kann.

Bevorzugt sind Oligonucleotidanaloga der Formel I sowie deren physiologisch verträglichen Salze, worin sich die Base B in β-Stellung befindet, die Nucleotide in der D-Konfiguration vorliegen und R² sich in 2'-Stellung befindet.

Insbesondere bevorzugt sind Oligonucleotidanaloga der Formel I, worin V, und Y die Bedeutung von Oxy haben.
Weiterhin besonders bevorzugt sind Oligonucleotidanaloga der Formel I, worin V, Y, und W die Bedeutung von Oxy bzw. Oxo haben.
Ganz besonders bevorzugt sind Oligonucleotidanaloga der Formel I, worin V, Y, W und U die Bedeutung von Oxy, Oxo bzw. Hydroxy haben.
Ferner sind Oligonucleotidanaloga der Formel I bevorzugt, worin R¹ für Wasserstoff steht.
Insbesondere bevorzugt sind Oligonucleotidanaloga der Formel I, worin U, V, W, X und Y die Bedeutung von Oxy, Oxo bzw. Hydroxy haben und R¹ gleich Wasserstoff ist.

Die wiederholt auftretenden Reste wie R², B, A, W, V, Y, U, R³, R⁴, T, a, b, p, q und Z können unabhängig voneinander gleiche oder verschiedene Bedeutungen haben, d.h. z.B. V bedeutet unabhängig voneinander Oxy, Sulfandiyl oder Imino.

Halogen steht vorzugsweise für Fluor, Chlor oder Brom.

Unter Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliederigen aromatischen Rings) durch S, NH oder O ersetzt sind. Desweiteren können auch ein oder beide Atome der Kondensationsstelle bicyclischer Reste (wie im Indolizinyl) N-Atome sein. Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Gruppen, die die intrazelluläre Aufnahme begünstigen, sind verschiedene lipophile Reste wie -O-(CH₂)ₓ-CH₃, worin x eine ganze Zahl von 6-18 bedeutet, -O-(CH₂)ₑ-CH=CH-(CH₂)_{f}-CH₃, worin e und f unabhängig voneinander eine ganze Zahl von 6 bis 12 bedeuten, -O-(CH₂CH₂O)₄-(CH₂)₉-CH₃, -O-(CH₂CH₂O)₈-(CH₂)₁₃-CH₃ und -O-(CH₂CH₂O)₇-(CH₂)₁₅-CH₃, aber auch Steroid-Reste wie Cholesteryl und Konjugate, die natürliche Carriersysteme ausnutzen wie Gallensäure, Folsäure, 2-(N-Alkyl-N-Alkoxy)-Aminoanthrachinon und Konjugate der Mannose und Peptide der entsprechenden Rezeptoren, die zur rezeptorvermittelten Endozytose der Oligonucleotide führen wie EGF (Epidermal Growth Factor), Bradykinin und PDGF (Platelet Derived Growth Factor). Unter Markierungs-Gruppen sind fluoreszierende Gruppen beispielsweise von Dansyl- (=N-Dimethyl-1-aminonaphthyl-5-sulfonyl-), Fluorescein- oder Coumarin-Derivaten oder chemilumineszierende Gruppen beispielsweise von Acridin-Drivaten zu verstehen sowie das über ELISA nachweisbare Digoxigenin-System, die über das Biotin/Avidin-System nachweisbare Biotin-Gruppe oder aber Linker- Arme mit funktionellen Gruppen, die eine nachträgliche Derivatisierung mit nachweisbaren Reporter-Gruppen gestatten, beispielsweise ein Aminoalkyl-Linker, der mit einem Acridinium-Aktivester zur Chemilumineszenz-Probe umgesetzt wird. Typische Markierungsgruppen sind: 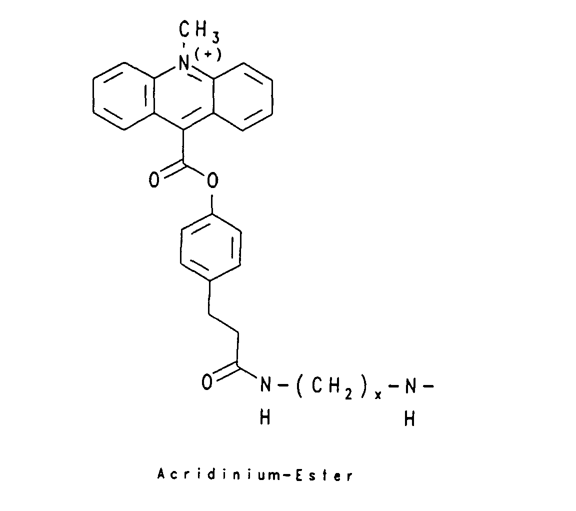 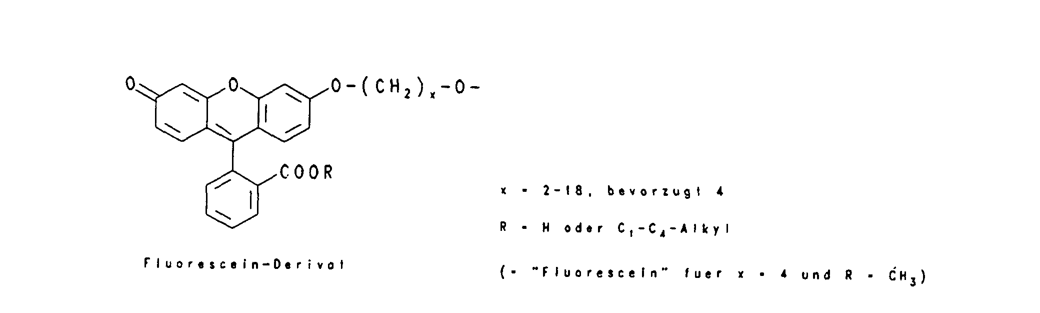 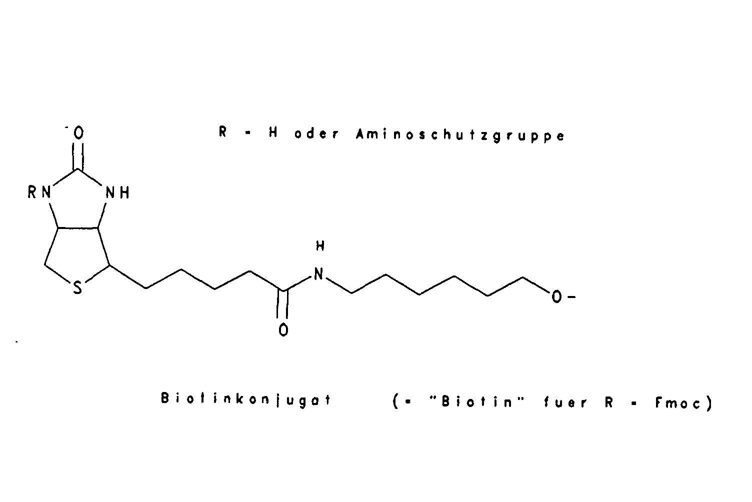 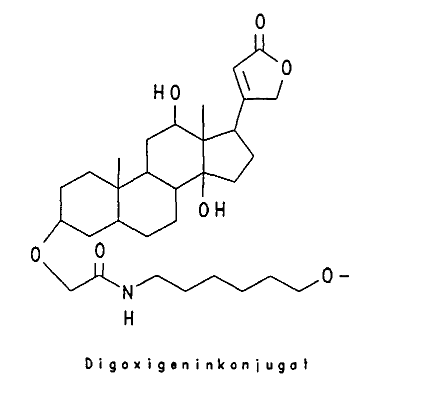 Oligonucleotidanaloga, die an Nucleinsäuren binden bzw. interkalieren und/oder spalten oder quervernetzen, enthalten Acridin-, Psoralen-, Phenanthrolin, Naphthochinon-, Daunomycin- oder Chlorethylaminoaryl-Konjugate. Typische interkalierende und quervernetzende Reste sind:

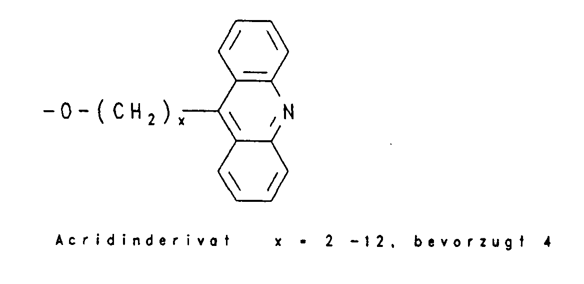

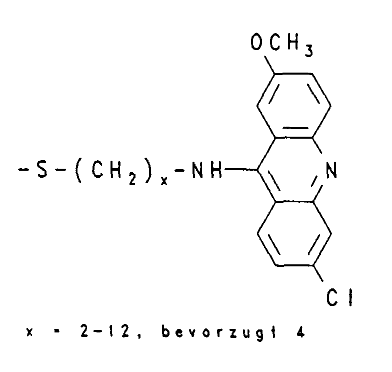

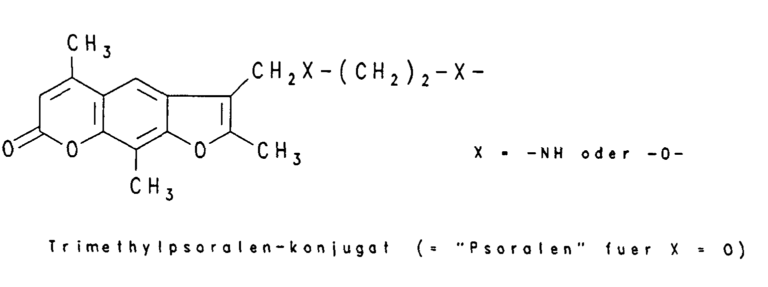

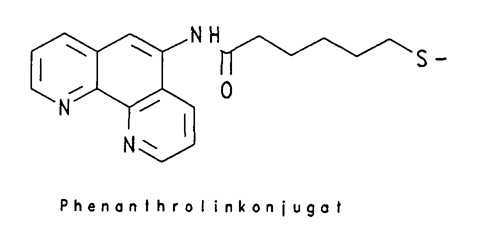

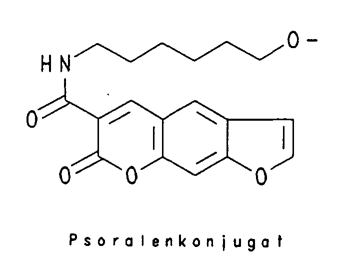

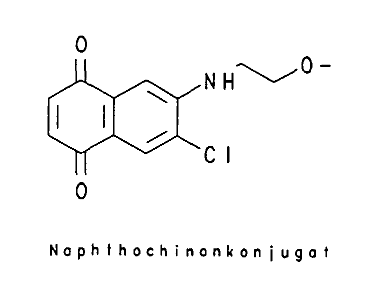

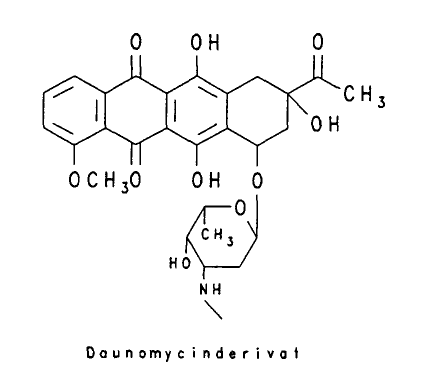

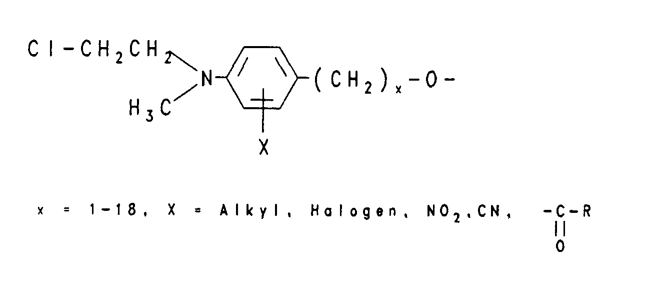

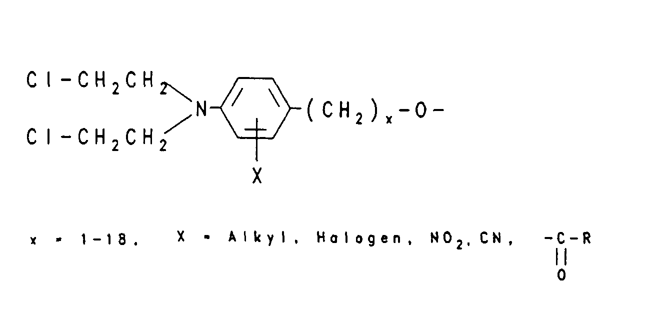

Unter physiologisch verträglichen Salzen von Verbindungen der Formel I versteht man sowohl anorganische als auch organische Salze, wie sie in Remington's Pharmaceutical Sciences (Mack Publ. Co., Easton, PA, 17. Auflage (1985) 1418) beschrieben sind. Aufgrund der physikalischen und chemischen Stabilität sind für saure Gruppen unter anderem Natrium-, Kalium-, Calcium und Ammoniumsalze bevorzugt.

Die Darstellung von Oligonucleotidanaloga der Formel I erfolgt nach bekannten Methoden analog der Synthese biologischer Oligonucleotide in Lösung oder vorzugsweise an fester Phase, gegebenenfalls unter Zuhilfenahme eines automatischen Synthesegeräts.

Es existieren verschiedene Methoden um Konjugat-Moleküle am 3'-Ende von Oligonucleotiden einzuführen. Diese liefern jedoch nicht Verbindungen der Formel I. Eine Übersicht über den Stand der Technik geben: M. Manoharan in Antisense Research and Applications, Crooke und Lebleu, Eds., Chapter 17, S. 303ff, CRC Press Boca Raton 1993, sowie EP-A 0 552 766 (HOE 92/F 012) und EP-A 0 552 767 (HOE 92/F 013). Während die Derivatisierung am 5'-Ende eines Oligonucleotids vergleichsweise einfach zu bewerkstelligen ist, beispielsweise durch Reaktion mit einem Phosphoramidit des entsprechenden Konjugatmoleküls unter Anwendung des Standard-Oligonucleotid-Synthesezyklus, existiert ein solches universell anwendbares Verfahren nicht für das 3'-Ende. Die 3'-Konjugation erfolgt entweder post-synthetisch - also nach der Abspaltung vom Träger bzw. nach Abspaltung der Schutzgruppen -, oder über ein, speziell für ein spezifisches Konjugatmolekül herzustellendes Trägermaterial. P. S. Nelson et al. (Nucl. Acids Res. 20 (1992) 6253) beschreiben einen 3'-Linker, von dem nach erfolgter Synthese am festen Träger alle Schutzgruppen abgespalten werden, dann post-synthetisch Konjugatmoleküle auf die freie Aminogruppe aufgekuppelt werden. Gamper et al (Nucl. Acids Res. 21 (1993) 145) beschreiben die Festphasensynthese mit Trägermaterial, welches mit dem einzuführenden Konjugatmolekül derivatisiert wurde. Für jedes Konjugatmolekül muß der Träger in aufwendiger Weise derivatisiert werden. In EP-A 0 552 766 und EP-A 0 552 767 wird ein β-eliminierbarer Linker beschrieben, auf welchen Nucleosidphosphoramidite aufgekuppelt werden, die anstelle der üblichen Cyanoethyl-Schutzgruppe das entsprechende Konjugatmolekül tragen. Danach erfolgt die Synthese des Oligonucleotids. Dies bedingt, daß das Konjugatmolekül keine säurelabile Schutzgruppe tragen darf, die im Verlauf des Synthesezyklus abgespalten würde. Außerdem ist die Synthese der Nucleosid-Konjugat Monomeren Bausteine sehr aufwendig.

Die erfindungsgemässen Verbindungen Können hergestellt werden durch ein universell einsetzbares Verfahren zur 3'-Modifikation von Oligonucleotiden am festen Träger, welches im Rahmen der Festphasensynthese die Einführung eines Konjugatmoleküls über Phosphoramidit-Chemie erlaubt. Für die Konjugation können die leicht zugänglichen, für die 5'-Derivatisierung gebräuchlichen Konjugat-Phosphoramidite eingesetzt werden. Das hierfür verwendete Linkermolekül mit den entsprechenden Schutzgruppen kann nicht nur am 3'-Ende des Oligonucleotids eingeführt werden, sondern auch ein- oder mehrfach innerhalb des Oligonucleotids unter Verwendung der Phosphoramidit-Chemie.

Das Verfahren zur Herstellung der Verbindungen der Formel I ist dadurch gekennzeichnet, daß man
a) eine Verbindung der Formel V 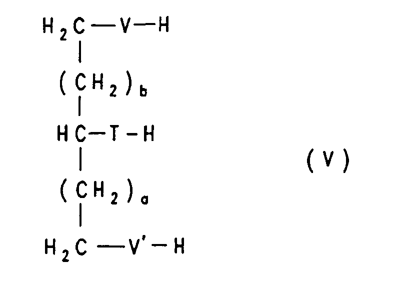 worin
   a, b, V, T wie oben in Formel I definiert sind und
   V' die Bedeutung von V hat und die funktionellen Gruppen V, V' und T auch gegebenenfalls temporär geschützt vorliegen können (vorzugsweise, falls V = V' = T = Oxy ist und b = 0, als cyclisches Acetal, das durch Umsetzung mit Aceton unter Fe^{III}-Katalyse erhalten wird und nach Einführung der Schutzgruppe S1 mit Essigsäure wieder abgespalten wird),
   mit einer Schutzgruppe S1, die sich von einem noch vollständig geschützten und am Träger gebundenem Oligonucleotid abspalten läßt, ohne daß andere Schutzgruppen oder die Bindung zum festen Träger gespalten werden, wie beispielsweise die Lävuloyl-Schutzgruppe, sowie ortho-, meta- oder para- R-O-Aryl-, wobei R für C₁-C₂₀-Alkyl, C₂-C₂₀-Alkenyl, C₃-C₂₀-Alkinyl, C₆-C₁₂-Aryl-C₁-C₆-alkyl steht, bevorzugt die Lävuloyl-Schutzgruppe und die para-Methoxyphenyl-Schutzgruppe,
   und einer Schutzgruppe S2, die sich ohne Spaltung des Linkerarmes Li in Formel VII und ohne Spaltung der Schutzgruppe S1 entfernen läßt, bevorzugt sind Dimethoxytrityl, Monomethoxytrityl, Trityl, Pixyl, 4-Methoxytetrahydropyranyl, besonders bevorzugt Monomethoxytrityl und Dimethoxytrityl,
   nach bekannten Verfahren (z.B. M. J. Gait, "Oligonucleotide Synthesis - a practical approach", IRL press 1984), beispielhaft wird die para-Methoxyphenylgruppe durch Umsetzung mit para-Methoxyphenol, Diphenylazodicarboxylat und Triphenylphosphin in einem geeigneten Lösungsmittel, z.B. Tetrahydrofuran (THF) unter Rückfluß eingeführt, danach das Acetal sauer, z.B. mit Essigsäure wieder abgespalten und anschließend die Monomethoxytrityl-Schutzgruppe durch Umsetzung mit Monomethoxytritylchlorid in Pyridin eingeführt, zu einer Verbindung der Formel VI umsetzt, 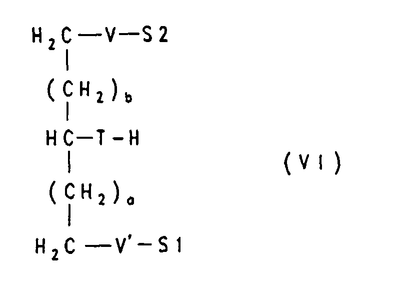 worin
   S1, S2, V, V', T, a und b wie oben definiert sind,
b) anschließend die Verbindung der Formel VI nach bekannten Verfahren mit 1 bis 10 Equivalenten, bevorzugt mit 1 bis 2 Equivalenten eines Linkers Li, wie beispielsweise Bernsteinsäureanhydrid, in einem geeigneten organischen Lösungsmittel wie z.B. Methylenchlorid, gegebenenfalls nach Zugabe eines Katalysators, beispielsweise 4-Dimethylaminopyridin, zu einer Verbindung der Formel VII umsetzt, 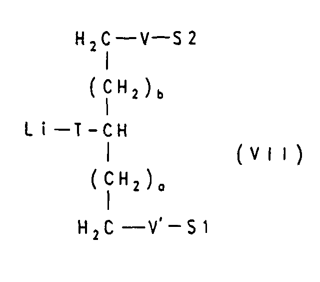 worin
   S1, S2, V, V', T, a und b wie oben definiert sind, und
   Li für einen Linkerarm steht, der durch chemische Bindung (Amid, Ester u.a.) die Verbindung der Formel VI mit einem festen Träger verbinden kann (Damka et al., Nucleic Acids Res. 18 (1990) 3813, Sonveaux (Bioorg. Chem. 14 (1986) 274), vorzugsweise ein Bernsteinsäurerest (O-C(O)-CH₂CH₂-C(O)-), ein Oxalsäurerest, (O-C(O)-C(O)-), ein Alkylamin, bevorzugt LCAA (Long Chain Alkylamin), oder Polyethylenglykol, besonders bevorzugt ein Bernsteinsäurerest, wobei in bestimmten Fällen, beispielsweise in Kombination mit Substituenten, die einer längeren Ammoniak-Behandlung nicht standhalten, auch labilere Linker, wie der Oxalyl-Linker, von Vorteil sind, und anschließend nach bekannten Verfahren, wie beispielsweise Extraktion, Kristallisation, Chromatographie aufarbeitet;
c) die Verbindung der Formel VII nach bekannten Verfahren auf einen festen Träger SS, wie beispielsweise Aminopropyl-CPG (CPG = Controlled Pore Glass) oder ®Tentagel (Fa. Rapp, Germany), beispielsweise durch Umsetzung mit DCC und p-Nitrophenol in einem geeigneten Lösungsmittel oder mit O-(Benzotriazol-1-yl)-N,N,N',N'-tetramethyluroniumtetrafluoroborat (TBTU) und einer Base, wie beispielsweise N-Ethylmorpholin, in einem geeigneten Lösungsmittel, wie z.B. DMF, koppelt (z.B. M.J. Gait, Oligonucleotide Synthesis - a practical approach, IRL Press, 1984) und man eine Verbindung der Formel VIII erhält, 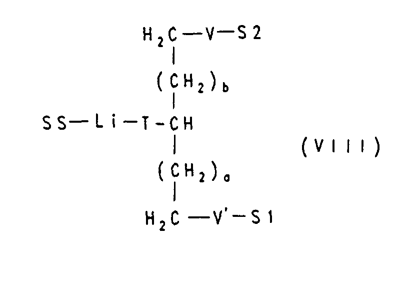 worin
   S1, S2, V, V', T, Li, a und b wie oben definiert sind und
   SS für den festen Träger steht, beispielsweise aus Materialien wie CPG (Controlled Pore Glass), Kieselgel oder einem organischen Harz wie Polystyrol (PS) oder einem Pfropf-Copolymer aus PS und Polyethylenglykol (POE), und durch funktionelle Gruppen wie Hydroxy, Amino, Halogen oder COOH in der Seitenkette modifiziert ist;
d) die Schutzgruppe S2 nach bekannten Verfahren, beispielsweise durch Behandlung mit 1-4% Dichloressigsäure (DCA) in Dichlormethan oder Chloroform abspaltet, oder
   alternativ zuvor die Schutzgruppe S1 nach bekannten Verfahren, z.B. die Lävuloyl-Schutzgruppe durch Behandlung mit Hydrazin, abspaltet, die Reaktionsschritte l) und m), dann die Reaktionsschritte e) - i) und anschließend Reaktionsschritt n) ausführt, oder
   alternativ dazu nach Abspaltung der Schutzgruppe S2 die Reaktionsschritte I) und m) ausführt, dann die Schutzgruppe S1 nach bekannten Verfahren, z.B. die Lävuloyl-Schutzgruppe durch Behandlung mit Hydrazin oder die para-Methoxyphenylschutzgruppe durch Behandlung mit Ce^{IV} abspaltet, danach die Reaktionsschritte e) - i) und schließlich Reaktionsschritt n) ausführt;
e) anschließend, falls m = 1 bis 5 ist, die nach d) erhaltene Verbindung mit einer Verbindung der Formel IX 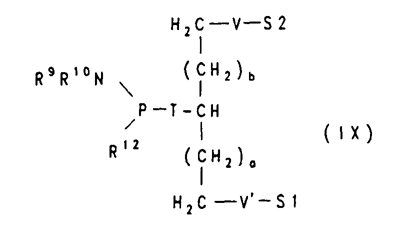 worin
   S1, S2, V, V', T, a und b wie oben definiert sind, und
   R⁹ und R¹⁰ gleich oder verschieden sind und C₁-C₈-Alkyl, vorzugsweise Isopropyl, oder C₅-C₁₂-Cycloalkyl, vorzugsweise bis C₈, Benzyl oder Phenyl oder zusammen mit dem Stickstoffatom, an das sie gebunden sind, einen gesättigten oder ungesättigten heterocyclischen Ring, mit gegebenenfalls weiteren Heteroatomen, wie z.B. Morpholin, und Substituenten, wie OC(O)O-C₁-C₄-AlkylEster, bedeuten,
   R¹² für OR¹³ oder C₁-C₁₈-Alkyl, C₁₋C₁₈-Alkoxy, C₆-C₂₀-Aryl, C₆-C₁₄-Aryl-C₁-C₈-Alkyl, bevorzugt für OR¹³, C₁-C₆-Alkyl, C₁-C₆-Alkoxy, C₆-C₂₀-Aryl, C₆-C₁₄-Aryl-C₁-C₈-Alkyl, besonders bevorzugt für OR¹³ oder C₁-C₆-Alkyl, steht,
   R¹³ für eine Gruppe der Formeln 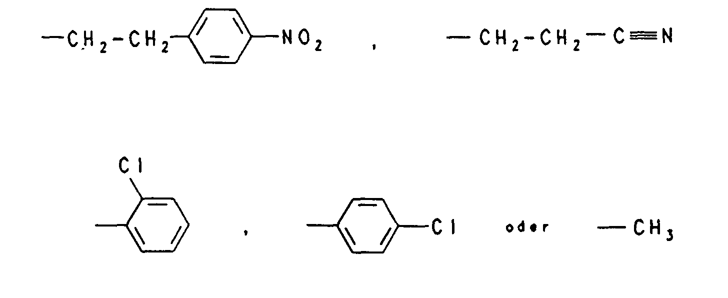 oder eine Benzylgruppe, welche nicht oder ein- bis vierfach ringsubstituiert, vorzugsweise nicht substituiert ist, steht, wobei der oder die Substituenten unabhängig voneinander Fluor, Chlor, Brom, eine C₁-C₄-Alkyl-, Nitro-, Methoxy-, oder Carboxylgruppe ist,
   in Gegenwart einer Verbindung der Formel [HNR¹⁴R¹⁵R¹⁶]⁽⁺⁾ E⁽⁻⁾ , wobei R¹⁴, R¹⁵, R¹⁶ gleich oder verschieden voneinander sind und eine C₁-C₄-Alkylgruppe und E = Fluor, Chlor, Brom, insbesondere Chlor, bedeuten, oder in Gegenwart von Tetrazol oder substituiertem Tetrazol, wie beispielsweise 5-(4-Nitrophenyl)-1H-tetrazol oder 5-Methylthio-1H-tetrazol oder 5-Ethylthio-1H-tetrazol, vorzugsweise in Gegenwart von substituiertem Tetrazol, wie beispielsweise 5-(4-Nitrophenyl)-1H-tetrazol oder 5-Methylthio-1H-tetrazol oder 5-Ethylthio-1H-tetrazol, besonders bevorzugt in Gegenwart von 5-Methylthio-1H-tetrazol, in einem geeigneten organischen Lösungsmittel, vorzugsweise Acetonitril umsetzt,
   die erhaltene Verbindung nach bekannten Verfahren oxidiert, z.B. wie in Reaktionsschritt m) beschrieben, ein Capping in der üblichen Weise durchführt, die Schutzgruppe S2 abspaltet, (z.B. Beaucage und Iyer Tetrahedron 49 (1993) 1925 & 2223 & 6123; E. Sonveaux, Bioorg. Chem. 14 (1986) 274; E. Uhlmann and A. Peyman, Chemical Reviews 90 (1990) 543)
   und gegebenenfalls diesen Reaktionsschritt noch (m-1)-mal wiederholt, wobei man eine Verbindung der Formel X erhält 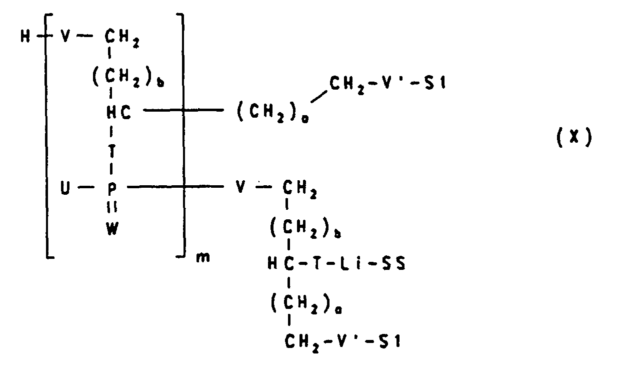 worin
   Li, S1, SS, T, U, V, V', W, a, b und m wie oben definiert sind;
f) falls m = 0 ist, die nach d) erhaltene Verbindung nach der Phosphoramidit-Methode (E. Sonveaux, Bioorg. Chem. 14 (1986) 274) mit einen Nucleosid-Phosphoramidit der Formel XI umsetzt, 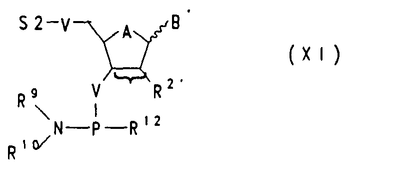 worin
   B' wie B und R^{2'} wie R² definiert sind und gegebenenfalls auch geschützt vorliegen können, z.B. kann R² = Hydroxy mit tert. Butyldimethylsilyl geschützt sein, und
   R⁹, R¹⁰, R¹², S2 und V wie oben definiert sind,
   die erhaltene Verbindung nach bekannten Verfahren oxidiert, ein Capping in der üblichen Weise durchführt, die Schutzgruppe S2, vorzugsweise Dimethoxytrityl oder Monomethoxytrityl, nach bekannten Verfahren abspaltet, (z.B. Beaucage und Iyer, Tetrahedron 49 (1993) 1925 & 2223 & 6123; E. Sonveaux, Bioorg. Chem. 14 (1986) 274; E. Uhlmann and A. Peyman, Chemical Reviews 90 (1990) 543)
   und gegebenenfalls diesen Reaktionsschritt noch (n-1)-mal wiederholt, wobei man eine Verbindung der Formel XII erhält 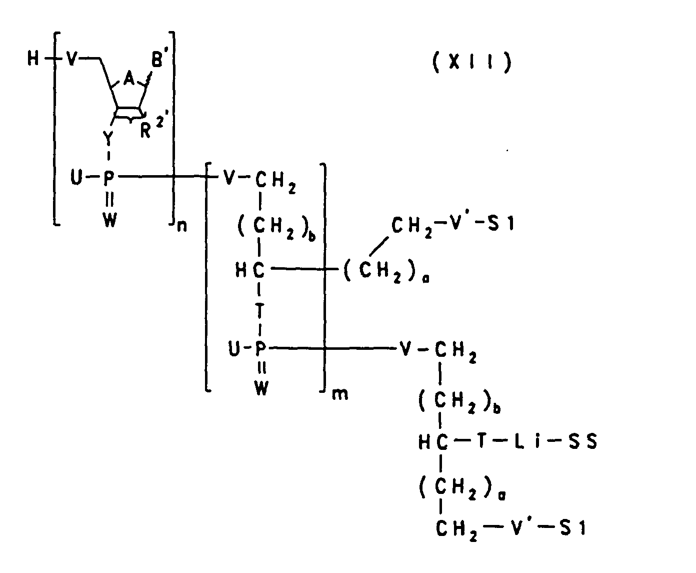 worin
   A, B', Li, R^{2'}, S1, SS, T, U, V, V', W, Y, a, b, m und n wie oben definiert sind;
g) falls m' = 1 bis 5, den Reaktionsschritt e) ausführt, der gegebenenfalls (m'-1)-mal wiederholt wird, wobei man die Verbindung der Formel XIII erhält, 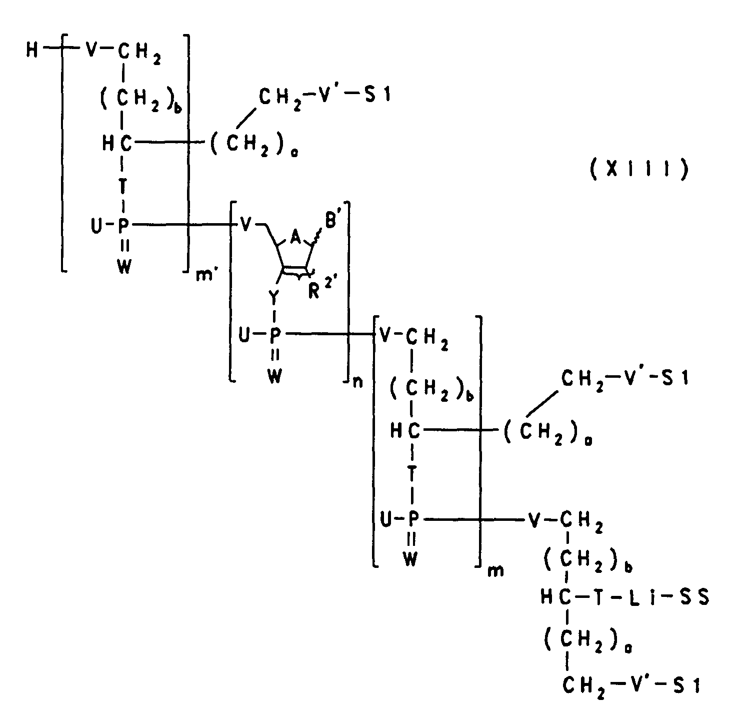 worin
   A, B', Li, R^{2'}, S1, SS, T, U, V, V', W, Y, a, b, m, m' und n wie oben definiert sind;
h) falls m' = 0 und n' = 1 - 50, den Reaktionsschritt f) ausführt, der gegebenenfalls noch (n'-1)-mal wiederholt wird, wobei man die Verbindungen der Formel XIV erhält, 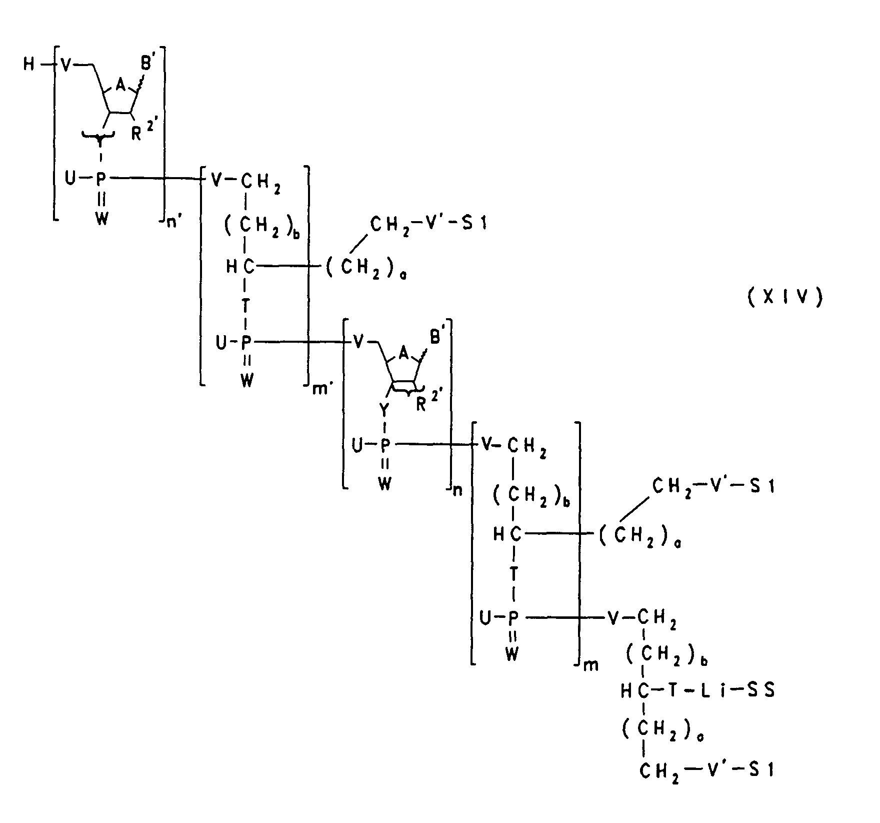 worin
   A, B', Li, R^{2'}, S1, SS, T, U, V, V', W, Y, a, b, m, m', n und n' wie oben definiert sind;
i) gegebenenfalls, falls in Formel I R¹ ≠ H, in der nach f), g) oder h) erhaltenen Verbindung den Rest R¹ nach bekannten Verfahren einführt, bevorzugt durch entsprechende Umsetzung analog den Reaktionsschritten l) und m),
   wobei
   R¹ C₁-C₁₈-Alkyl, vorzugsweise C₁-C₆-Alkyl, insbesondere Methyl, C₂-C₁₈-Alkenyl, C₃-C₁₈-Alkinyl, C₁-C₁₈-Alkylcarbonyl, C₂-C₁₉ Alkenylcarbonyl, C₃-C₁₉-Alkinylcarbonyl, C₆-C₂₀-Aryl, C₆-C₁₄-Aryl-C₁-C₈-alkyl, oder einen Rest der Formel III 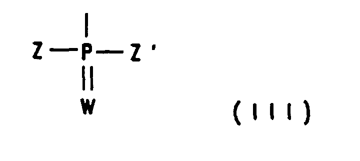 worin
   W, Z und Z' wie oben definiert sind, bedeuten,
   bevorzugt einen Rest der Formel III;
j) falls in Formel I R¹ = H, nach bekannten Methoden cappt, z.B. durch Umsetzung mit Acetanhydrid und N-Methylimidazol;
k) anschließend von den so erhaltenen, noch trägergebundenen, geschützten Oligonucleotiden die Schutzgruppe S1 nach bekannten Verfahren (z.B. Greene, Wuts, "Protective Groups in Organic Synthesis", J. Wiley, New York 1991) abspaltet, so daß der Linker zum festen Träger und die anderen im Molekül enthaltenen Schutzgruppen erhalten bleiben, beispielsweise für S1 = Lävuloyl durch Behandlung mit Hydrazin und für S1 = para-Methoxyphenyl vorzugsweise durch Behandlung mit Ce^{IV}, beispielsweise mit einer 0,05 - 1M Lösung von Ce^{IV}(NH₄)₂(NO₃)₆ in Acetonitril/H₂O bei -10 bis 100°C während 0,2 bis 500 Minuten, bevorzugt mit einer 0,05 bis 0,5M, insbesondere 0,1M Lösung von Ce^{IV}(NH₄)₂(NO₃)₆ in Acetonitril/H₂O (2:1 bis 8:1, insbesondere 4:1) bei 0-50 °C, insbesondere 20-30 °C während 1-30 min, insbesondere während 2 bis 10 min;
l) und die so erhaltene Verbindung mit einer Verbindung der Formel XV umsetzt, 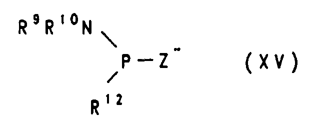 worin
   R⁹, R¹⁰, R¹² die oben genannten Bedeutungen haben und
   Z'' die Bedeutung von Z hat, das wie oben definiert ist, oder auch für nach bekannten Verfahren geschütztes Z steht, wobei vorzugsweise Schutzgruppen Anwendung finden, die unter Bedingungen abgespalten werden, die bei der Oligonucleotid-Synthese zur Schutzgruppen-Abspaltung zur Anwendung kommen, beispielhaft seien Hydroxy, Mercapto und SeH genannt, die als geschützte Derivate vorliegen müssen, beispielsweise als O-CH₂-CH₂-CN, O-CH₃, S-CH₂-CH₂-CN oder 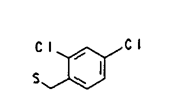 in Gegenwart einer Verbindung der Formel [HNR¹⁴R¹⁵R¹⁶]⁽⁺⁾ E⁽⁻⁾, wobei R¹⁴, R¹⁵, R¹⁶ und E wie oben definiert sind, oder in Gegenwart von Tetrazol oder substituiertem Tetrazol, wie beispielsweise 5-(4-Nitrophenyl)-1H-tetrazol oder 5-Methylthio-1H-tetrazol oder 5-Ethylthio-1H-tetrazol, vorzugsweise in Gegenwart von substituiertem Tetrazol, wie beispielsweise 5-(4-Nitrophenyl)-1H-tetrazol oder 5-Methylthio-1H-tetrazol oder 5-Ethylthio-1H-tetrazol, besonders bevorzugt in Gegenwart von 5-Methylthio-1H-tetrazol, in einem geeigneten organischen Lösungsmittel, vorzugsweise Acetonitril;
m) die erhaltene Verbindung nach bekannten Verfahren oxidiert, beispielsweise durch Umsetzung mit Jod in Gegenwart von wäßrigem Pyridin, Lutidin oder Collidin, gegebenenfalls auch in Gegenwart weiterer organischer Lösungsmittel wie beispielsweise Tetrahydrofuran, oder beispielsweise durch Umsetzung mit N,N,N',N'-Tetraethylthiuramdisulfid in Acetonitril, oder beispielsweise durch Umsetzung mit Jod in Gegenwart von Alkylamin oder Arylamin, wobei die verschiedenen, dem Fachmann bekannten Oxidationsverfahren, die der Herstellung von natürlichen und modifizierten Oligonucleotiden dienen, beispielsweise in Beaucage und Iyer, Tetrahedron 49 (1993) 1925 & 2223 & 6123; E. Sonveaux, Bioorg. Chem. 14 (1986) 274 sowie E. Uhlmann and A. Peyman, Chemical Reviews 90 (1990) 543 zusammengefaßt sind, und die Oxidation bevorzugt durch Umsetzung mit Jod in Gegenwart von wäßrigem Pyridin, Lutidin oder Collidin, gegebenenfalls auch in Gegenwart weiterer organischer Lösungsmittel wie Tetrahydrofuran erfolgt;
n) das Oligonucleotid nach bekannten Verfahren vom Träger abspaltet, beispielhaft mit NH₃ bei 50 - 60 °C, und die verbliebenen Schutzgruppen am Phosphat und Nucleobasen ebenfalls nach bekannten Verfahren abspaltet.

Die Art der Amino-Schutzgruppen der Basen und die Beschaffenheit des Linkers Li richten sich im Einzelfall nach der Art des Substituenten Z, da diese nach vollständiger Synthese problemlos spaltbar sein müssen. Zum Beispiel kann man bei der Herstellung eines Oligonucleotid-3'-Phosphatisopropylesters (Z=O-i-C₃H₇), für B = Ade und Cyt mit den Benzoyl- (Bz) bzw. für B = Gua mit den Isobutyryl- (i-Bu) Schutzgruppen arbeiten. Dagegen verwendet man zur Synthese eines Oligonucleotid-3'- methylphosphonats (Z = CH₃) oder Ethylesters (Z = O-C₂H₅) für B = Ade und Gua bevorzugt die labileren Phenoxyacetyl (PAC) bzw. für B = Cyt die iso-Butyryl-Schutzgruppen.

Die Verbindungen der Formel IX 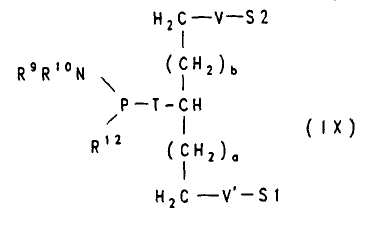 worin
S1, S2, V, V', T, a, und b, wie oben definiert sind,
R⁹ und R¹⁰ gleich oder verschieden sind und C₁-C₈-Alkyl, vorzugsweise Isopropyl, oder C₅-C₁₂-Cycloalkyl, vorzugsweise bis C₈, Benzyl oder Phenyl oder zusammen mit dem Stickstoffatom, an das sie gebunden sind, einen gesättigten oder ungesättigten heterocyclischen Ring, mit gegebenenfalls weiteren Heteroatomen, wie z.B. Morpholin, und Substituenten, wie OC(O)O-C₁-C₄-Alkyl-Ester, bedeuten; und
R¹² für OR¹³ oder C₁-C₁₈-Alkyl, C₁-C₁₈-Alkoxy, C₆-C₂₀-Aryl, C₆-C₁₄-Aryl-C₁-C₈-Alkyl, bevorzugt für OR¹³, C₁-C₆-Alkyl, C₁-C₆-Alkoxy, C₆-C₂₀-Aryl, C₆-C₁₄-Aryl-C₁-C₈-Alkyl, besonders 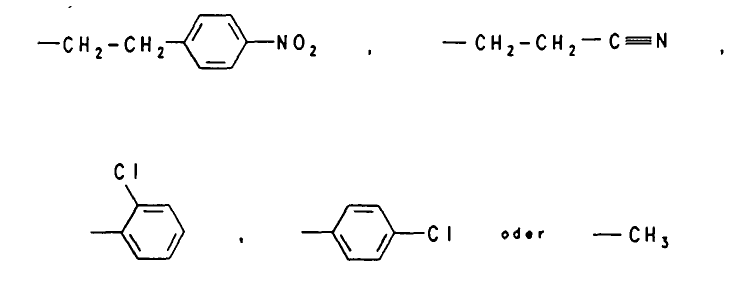 bevorzugt für OR¹³ oder C₁-C₆-Alkyl, steht;
R¹³ für eine Gruppe der Formel oder eine Benzylgruppe, welche nicht oder ein- bis vierfach ringsubstituiert, vorzugsweise nicht substituiert ist, wobei der oder die Substituenten unabhängig voneinander Fluor, Chlor, Brom, eine C₁-C₄-Alkyl-, Nitro-, Methoxy-, oder Carboxylgruppe ist, steht,
können erhalten werden durch Umsetzung einer Verbindung der Formel VI 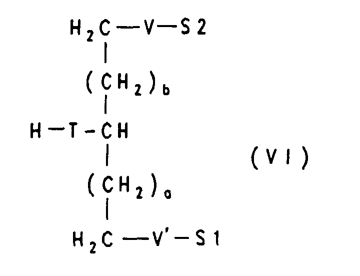 mit einer Verbindung der Formel XX 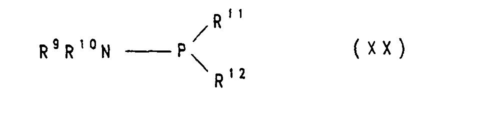 worin
R⁹, R¹⁰ und R¹² wie oben definiert sind und
R¹¹ für Chlor oder Brom oder einen Rest der Formel NR⁹R¹⁰ steht, wobei R⁹ und R¹⁰ wie oben definiert sind;
in Gegenwart einer Base, vorzugsweise Pyridin oder einer Mischung von Tetrahydrofuran (THF), Dioxan, Dichlormethan (DCM), Chloroform, und/oder Acetonitril mit einem C₁-C₄-Trialkylamin, vorzugsweise Trimethyl-, Triethyl- oder Diisopropylethyl-Amin, oder, wenn R¹¹ ein Rest der Formel NR⁹R¹⁰ ist, dann in Gegenwart einer Verbindung der Formel [HNR¹⁴R¹⁵R¹⁶]⁽⁺⁾ E⁽⁻⁾, wobei R¹⁴, R¹⁵, R¹⁶ gleich oder verschieden voneinander sind und eine C₁-C₄-Alkylgruppe und E = Fluor, Chlor, Brom, insbesondere Chlor, bedeuten, oder in Gegenwart von Tetrazol oder substituiertem Tetrazol, wie beispielsweise 5-(4-Nitrophenyl)-1H-tetrazol oder 5-Methylthio-1H-tetrazol oder 5-Ethylthio-1H-tetrazol, vorzugsweise in Gegenwart von Tetrazol.

Anstelle der Phosphoramidit-Methode können die Verbindungen der Formel I auch mittels Festphasensynthese nach der H-Phosphonat-Methode oder der Phosphotriester-Methode (E. Uhlmann and A. Peyman, Chemical Reviews 90 (1990) 543) erhalten werden.

Bei Verwendung der H-Phosphonat-Methode wird die nach dem Reaktionsschritt a) (Herstellung von Verbindungen der Formel I) erhaltene Verbindung der Formel VI nach bekannten Verfahren (z.B. B. Froehler, Tetrahedron Lett. 27 (1986) 5575) zu einer Verbindung der Formel XXI umgesetzt, 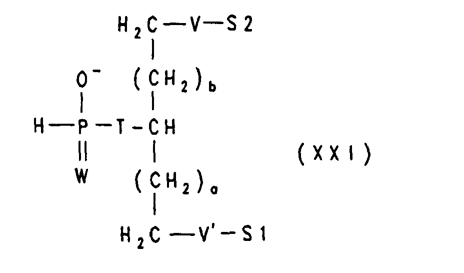 worin V, V', T, a, b, und W die oben genannte Bedeutung haben. Beispielhaft sei genannt die Umsetzung mit 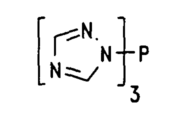 in einem geeigneten organischen Lösungsmittel, z. B. Dichlormethan und anschließender Hydrolyse. Bei Einführung der Gruppe Z (Reaktionsschritt i) bei Verbindungen der Formel I und Reaktionsschritt e) bei Verbindungen der Formel II) nach der H-Phosphonat-Methode wird mit einer Verbindung der Formel XXII 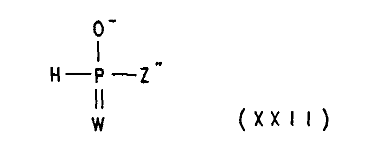 worin Z" und W die oben genannten Bedeutungen haben,
in Gegenwart eines Kondensationsmittels, wie Pivaloyl- oder Adamantoylchlorid, und einer Base, wie Pyridin umgesetzt. Der gebildete H-Phosphonat-Diester wird dann einer oxidativen Phosphoramidierung (B. Froehler, Tetrahedron Lett. 27, (1986) 5575) bzw. einer Jodwasser-, Schwefel- oder Selenoxidation unterworfen. Auf diese Weise läßt sich zum Beispiel mit Cholesteryloxycarbonyl-aminoalkylamin in Gegenwart von Tetrachlorkohlenstoff ein Oligonucleotid mit einer 3'-terminalen Cholesteryl-Gruppe herstellen. Durch oxidative Amidierung mit 2-Methoxyethylamin erhält man beispielsweise Oligonucleotide mit einem 3'-O-(2-Methoxyethyl)-phosphoramidat-Rest.

Nach der Triester-Methode wird die nach Reaktionschritt a) (Herstellung von Verbindungen der Formel I) erhaltene Verbindung der Formel VI nach bekannten Verfahren (z.B. Sonveaux, Bioorg. Chem. 14 (1986) 274) zu einer Verbindung der Formel XXIII umgesetzt 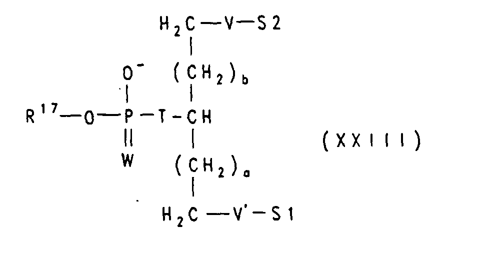 worin V, V', T, a, b, und W die oben genannte Bedeutung haben, und R¹⁷ für eine der dem Fachmann bekannten im Triester-Verfahren gebräuchlichen Schutzgruppen steht, z.B. 2,4-Dichlorphenyl ( E. Sonveaux, Bioorg. Chem. 14 (1986) 274) steht. Bei Einführung der Gruppe Z (Reaktionsschritt i) bei Verbindungen der Formel I und Reaktionsschritt e) bei Verbindungen der Formel II) nach der Triester-Methode wird mit einer Verbindung der Formel XXIV, 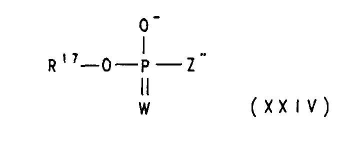 worin Z, W und R¹⁷ wie oben definiert sind, in Gegenwart eines Kondensationsmittels umsetzt. Bevorzugte Kondensationsreagenzien sind Arylsulfonsäurechloride wie Mesitylen-, 2.4.6-Triisopropylbenzol- oder 8-Chinolinsulfonsäurechlorid in Gegenwart nucleophiler Katalysatoren wie Imidazol, Triazol, Tetrazol oder deren substituierte Derivate wie N-Methylimidazol, 3-Nitrotriazol oder 5-(p-Nitrophenyl)-tetrazol. Besonders bevorzugte Kondensationsmittel sind 4-substituierte Derivate von Pyridin-N-oxid oder Chinolin-N-oxid (Efimov et al., Nucleic Acids Research 13 (1985) 3651).

Oligonucleotidanaloga der Formel I finden als Inhibitoren der Genexpression Verwendung.

Die Verbindungen der vorliegenden Erfindung können beispielsweise als Arzneimittel bei der Behandlung von Erkrankungen, die durch Viren (HIV, HSV-1, HSV-2, Influenza, VSV, Hepatitis B oder Papilloma Viren) hervorgerufen werden, verwendet werden.

Antisense Oligonucleotid-Sequenzen, erfindungsgemäß modifiziert, die gegen solche Targets wirksam sind, sind beispielsweise:
a) gegen HIV, z. B. 5′-A C A C C C A A T T C T G A A A A T G G -3′ (I) oder 5′-A G G T C C C T G T T C G G G C G C C A-3′ (II) oder 5′-G T C G A C A C C C A A T T C T G A A A A T G G A T A A-3′ (III) oder 5'-G C T A T G T C G A C A C C C A A T T C T G A A A-3'    (IV) oder 5′-T C G T C G C T G T C T C C G C T T C T T C T T C C T G C C A (V) oder 
5'-C T G T C T C C G C T T C T T C T T C C T G C C A T A G G A G -3'
(VI)
 oder
b) gegen HSV-1, z.B.

5'-G C G G G G C T C C A T G G G G G T C G-3'          (VII)

Die Verbindungen der vorliegenden Erfindung eignen sich beispielsweise auch zur Behandlung von Krebs. Beispielsweise können dabei Oligonucleotid-Sequenzen zum Einsatz kommen, die gegen Targets gerichtet sind, die für Krebsentstehung bzw. Krebswachstum verantwortlich sind. Weiterhin eignen sich Arzneimittel der vorliegenden Erfindung beispielsweise auch zur Verhinderung der Restenose. Beispielsweise können dabei Oligonucleotidsequenzen zum Einsatz kommen, die gegen Targets gerichtet sind, welche für die Proliferation oder Migration verantwortlich sind. Solche Targets sind beispielsweise:
1) Nucleare Onkoproteine, wie beispielsweise c-myc, N-myc, c-myb, c-fos, c-fos/jun, PCNA, p120
2) Cytoplasmische/Membran-assoziierte Onkoproteine, wie beispielsweise EJ-ras, c-Ha-ras, N-ras, rrg, bcl-2, cdc-2, c-raf-1 , c-mos, c-src, c-abl
3) Zelluläre Rezeptoren, wie beispielsweise EGF-Rezeptor, FGF-Rezeptor, c-erbA, Retinoid-Rezeptoren, Protein-Kinase regulatorische Untereinheit, c-fms, cdc2 kinase,
4) Cytokine, Wachstumsfaktoren, Extrazelluläre Matrix, wie beispielsweise CSF-1, IL-6, IL-1a, IL-1b, IL-2, IL-4, bFGF, IGF, Myeloblastin, Fibronectin.

Antisense Oligonucleotid-Sequenzen, erfindungsgemäß modifiziert, die gegen solche Targets wirksam sind, sind beispielsweise
a) gegen c-Ha-ras, z.B. 5′- C A G C T G C A A C C C A G C -3′ (VIII) oder
c) c-myc, z.B. 5'- G G C T G C T G G A G C G G G G C A C A C-3'     (IX) oder 5′-A A C G T T G A G G G G C A T-3′ (X) oder
d) c-myb, z.B. 5′-G T G C C G G G G T C T T C G G G C -3′ (XI) oder
e) c-fos, z.B. 5'-G G A G A A C A T C A T G G T C G A A A G-3'     (XII) oder 5'-C C C G A G A A C A T C A T G G T C G A A G-3'    (XIII) oder 5′-G G G G A A A G C C C G G C A A G G G G-3′ (XIV) oder
f) p120, z.B. 5′-C A C C C G C C T T G G C C T C C C A C-3′ (XV) oder
g) EGF-Rezeptor, z.B. 5'-G G G A C T C C G G C G C A G C G C -3'     (XVI) oder 5'-G G C A A A C T T T C T T T T C C T C C-3'          (XVII) oder
h) p53 Tumorsuppressor, z.B. 5'- G G G A A G G A G G A G G A T G A G G-3'    (XVIII) oder

5′-G G C A G T C A T C C A G C T T C G G A G-3′ (XIX)

Die Verbindungen der vorliegenden Erfindung eignen sich beispielsweise ferner zur Behandlung von Erkrankungen, die durch Integrine oder Zell-Zell-Adhäsionsrezeptoren beeinflußt werden, beispielsweise durch VLA-4, VLA-2, ICAM oder ELAM.

Antisense Oligonucleotid-Sequenzen, erfindungsgemäß modifiziert, die gegen solche Targets wirksam sind, sind beispielsweise
a) VLA-4, z.B. 5′-G C A G T A A G C A T C C A T A T C -3′ (XX) oder
b) ICAM, z.B. 5'- C C C C C A C C A C T T C C C C T C T C-3'     (XXI) oder 5'-C T C C C C C A C C A C T T C C C C T C-3'     (XXII) oder 5′-G C T G G G A G C C A T A G C G A G G-3′ (XXIII) oder
c) ELAM-1, z. B.

5′-A C T G C T G C C T C T T G T C T C A G G -3′ (XXIV)

Ferner können die Oligonucleotidanaloga der Formel I als Sonde zum Nachweis von Nucleinsäuren oder als Hilfsmittel in der Molekularbiologie dienen.

Weitere Gegenstände der Erfindung sind pharmazeutische Zubereitungen enthaltend ein oder mehrere Oligonucleotidanaloga der Formel I gegebenenfalls zusammen mit physiologisch verträglichen Hilfs- und/oder Trägerstoffen und/oder zusammen mit anderen bekannten Wirkstoffen sowie Verfahren zu deren Herstellung.

### Beispiele

### 1) Synthese von (4-Methoxyphenyl)-6-O-(4-methoxytriphenylmethyl)-5-O-succinylhexylether

### 1a) 2,2-Dimethyl-4-hydroxybutyl-1,3-dioxolan

15g (112mmol) 1,2,6-Hexantriol wurden zusammen mit 0,5g FeCl₃ in 1l Aceton gelöst und 7h unter Rückfluß gekocht. Es wurde filtriert und überschüssiges Aceton abdestilliert, wobei das Produkt in reiner Form erhalten wurde.
Ausbeute: 18,8g (96%);
¹H-NMR (200MHz, CDCl₃/TMS): d = 1,35 (s, 3H, CH₃); 1,40 (s, 3H, CH₃); 1,30-1,40 (m, 6H, -(CH₂)₃-); 3,52 (t, 1H, C⁴-H); 3,68 (t, 2H, CH₂-OH); 4,00-4,20 (m, 2H, -C⁵H₂-);
MS (El): m/e = 175 (M+H⁺, 50%); 159 (30%)

### 1b) (4-Methoxyphenyl)-4-(2,2-dimethyl)dioxolan-4-yl)butylether

1,74g (10mmol) 2,2-Dimethyl-4-hydroxybutyl-1,3-dioxolan aus Beispiel 1a, 3,41g (13mmol) Triphenylphosphin, 2,26g (13mmol) Diethylazodicarboxylat und 3,72g (30mmol) 4-Methoxyphenol wurden in 30 ml absol. Tetrahydrofuran (THF) gelöst und für 1h unter Rückfluß gekocht. Das Lösungsmittel wurde abdestilliert und der Rückstand über Kieselgel mit Essigsäureethylester (EE)/n-Heptan (1:4) chromatographiert.
Ausbeute: 2,1g (74%);
¹H-NMR (200MHz, CDCl₃/TMS): d = 1,35 (s, 3H, CH₃); 1,41 (s, 3H, CH₃); 1,45-1,90 (m, 6H, -(CH₂)₃-); 3,53 (t, 1H, C^{4'}-H); 3,77 (s, 3H, O-CH₃); 3,92 (t, 2H, CH₂-OAr); 3,99-4,21 (m, 2H, -C^{5'}H₂-); 6,84 (s, 4H, Ar-H);
MS (El): m/e = 280 (M+H⁺, 90%); 265 (50%); 223 (100%)

### 1c) (4-Methoxyphenyl)-(5,6-dihydroxy)hexylether

2,08g (4-Methoxyphenyl)-4-(2,2-dimethyldioxolan-4yl)butylether aus Beispiel 1b wurden in 165ml 80% Essigsäure gelöst und 4h bei Raumtemperatur gerührt. Die Essigsäure wurde im Vakuum abgedampft, es wurde noch zweimal mit Toluol/Methanol koevaporiert. Dabei fiel das Produkt kristallin an.
Ausbeute: 1,15g (65%), mp: 69 °C
¹H-NMR (200MHz, CDCl₃/TMS): d = 1,40-1,91 (m, 6H, -(CH₂)₃-); 3,39-3,52 (m, 1H, C⁵-H); 3,42-3,74 (m, 2H, C⁶H₂); 3,77 (s, 3H, O-CH₃); 3,93 (t, 2H, CH₂-OAr); 6,82 (s, 4H, Ar-H);
MS (EI): m/e = 241 (M+H⁺, 60%); 240 (M⁺, 100%) 223 (30%), 205 (30%)

### 1d) (4-Methoxyphenyl)-6-O-(4-methoxytriphenylmethyl)-5-hydroxy-hexylether

1,96g (8,2 mmol) (4-Methoxyphenyl)-5,6-dihydroxy-hexylether aus Beispiel 1c und 2,78g (9,0 mmol) 4-Methoxytriphenylmethylchlorid wurden in 30 ml absol. Pyridin gelöst und 3h bei Raumtemperatur gerührt. Das Pyridin wurde im Vakuum abgedampft, der Rückstand in 40 ml Dichlormethan (DCM) aufgenommen und erst mit 40ml 5% NaHCO₃-Lösung, dann mit 40ml gesättigter NaCl-Lösung extrahiert und zweimal mit Wasser gewaschen. Es wurde über Natriumsulfat getrocknet, das Lösungsmittel abdestilliert, der Rückstand mit EE/n-Heptan (1:2) über Kieselgel chromatographiert.
Ausbeute: 3,10g (74%);
¹H-NMR (200MHz, CDCl₃/TMS): d = 1,37-1,80 (m, 6H, -(CH₂)₃-); 2,30 (d, J = 5Hz, 1H, C⁵-H); 3,00-3,23 (m, 2H, CH₂-OMMTr); 3,73 (s, 3H, O-CH₃); 3,80 (s, 3H, O-CH₃); 3,88 (t, 2H, CH₂-OAr); 6,80 (s, 4H, Ar-H); 7,15-7,47 (m, 14H, Ar-H);
MS (ES⁺, +LiCl): m/e = 519 (M+Li⁺, 100%);

### 1e) (4-Methoxyphenyl)-6-O-(4-methoxytriphenylmethyl)-5-O-succinylhexylether

3,1 g (6,05 mmol) (4-Methoxyphenyl)-6-O-(4-methoxytriphenylmethyl)-5-hydroxyhexylether aus Beispiel 1d wurden zusammen mit 0,85g (8,47mmol) Bernsteinsäureanhydrid und 1,04g (8,47mmol) N,N-Dimethylaminopyridin (DMAP) in 20ml absol. Pyridin gelöst und 19h bei Raumtemperatur gerührt. Das Lösungsmittel wurde im Vakuum abgedampft. Es wurde noch zweimal mit Toluol/Methanol koevaporiert, der Rückstand in 280ml DCM aufgenommen und mit 140 ml 10% Zitronensäure, dann zweimal mit Wasser gewaschen und über Natriumsulfat getrocknet. Das Lösungsmittel wurde abdestilliert, der Rückstand mit EE/n-Heptan 2:1 über Kieselgel chromatographiert.
Ausbeute: 2,55g (69%);
¹H-NMR (200MHz, CDCl₃/TMS): d = 1,27-1,49 (m, 2H, C²H₂); 1,60-1,82 (m, 4H, C¹H₂ & C³H₂); 2,66 (s, 4H, CO-(CH₂)₂-CO); 3,15 (d, 2H, CH₂-OMMTr); 3,75 (s, 3H, O-CH₃); 3,78 (s, 3H, O-CH₃); 3,89 (t, 2H, CH₂-OAr); 5,12 (dt, 1H, CH-Osucc); 6,80 (s, 4H, Ar-H); 7,11-7,52 (m, 14H, Ar-H);
MS (FAB+LiCl): m/e = 625.3 (M+2Li⁺-H⁺, 100%); 619.2 (M+Li⁺, 70%); 612.2 (M⁺, 100%)

### 2) Synthese von (4-Methoxyphenyl)-6-O-(4-methoxytriphenylmethyl)-5-O-phosphorigsäure-β-cyanoethylester-N,N-diisopropylamidhexylether

512 mg (1,0 mmol) (4-Methoxyphenyl)-1-(6-O-(4-methoxytriphenylmethyl)-5-hydroxy)hexylether aus Beispiel 1d wurden nach koevaporieren zusammen mit 390 mg (3,0 mmol) Diisopropylethylamin mit absol. Acetonitril in 4 ml absol. THF gelöst. Unter Schutzgas wurden 330 mg (1,4 mmol) N,N-Diisopropyl-cyanoethyl-phosphoramidochlorid langsam zugetropft. Es wurde 2h bei Raumtemperatur gerührt. Das Lösungsmittel wurde abgedampft, der Rückstand in 20 ml EE aufgenommen und mit 40 ml gesättigter NaCl-Lösung extrahiert. Die organische Phase wurde noch zweimal mit Wasser gewaschen, dann über Natriumsulfat getrocknet. Das Lösungsmittel wurde abdestilliert, der Rückstand mit DCM/Ethanol/Triethylamin (TEA) (100:4:2) über Kieselgel chromatographiert.
Ausbeute: 520 mg;
¹H-NMR (200MHz, CDCl₃/TMS): d = 1,00-1,93 (m, 18H, -(CH₂)₃- & 4x CH₃); 2,38 & 2,57 (jew.: t, 1H, CH₂-CN); 2,92-3,26 (m, 2H, P-O-CH₂), 3,45-4,20 (m, 13H, 2 x OCH₃ & 2 x CH(CH₃)₂ & CH₂-OAr & CH₂-O-MMTr & C⁵H); 6,70-6,87 (s, 4H, Ar-H); 7,14-7,32 (m, 14H, Ar-H);
MS (FAB, LiCl; NBA): m/e = 735.5 (M+Na⁺, 100%); 719.5 (M+Li⁺, 50%)

### 3) Synthese von (4-Methoxyphenyl)-3-O-(4-methoxytriphenylmethyl)-2-O-succinylpropylether

### 3a) (4-Methoxyphenyl)-(2,2-dimethyldioxolan-4-yl)methylether

Die Synthese erfolgte analog Beispiel 1b aus 2,2-Dimethyl-4-hydroxymethyl-1,3-dioxolan.
Ausbeute: 56%;
¹H-NMR (200MHz, CDCl₃/TMS): d = 1,40 (s, 3H, CH₃); 1,44 (s, 3H, CH₃); 3,78 (s, 3H, O-CH₃); 3,89 (dd, 2H, CH₂-OAr); 3,97-4,21 (m, 2H, -C³H₂-); 4,45 (dt, 1H, C²H); 6,83 (s, 4H, Ar-H);
MS (EI): m/e = 239 (M+H⁺, 40%); 238 (M⁺, 50%)

### 3b) (4-Methoxyphenyl)-2,3-dihydroxypropylether

Die Synthese erfolgte analog Beispiel 1c aus (4-Methoxyphenyl)-(2,2-dimethyldioxolan-4yl)methylether (Beispiel 3a).
Ausbeute: 98%;
MS (El): m/e = 199 (M+H⁺, 100%); 198 (M⁺, 80%); 181 (40%); 163 (70%)

### 3c) (4-Methoxyphenyl)-3-O-(4-methoxytriphenylmethyl)-2-hydroxypropylether

Die Synthese erfolgte analog Beispiel 1d aus (4-Methoxyphenyl)-2,3-dihydroxypropylether (Beispiel 3b).
Ausbeute: 46%;
¹H-NMR (200MHz, CDCl₃/TMS): d = 3,3,31 (d, 2H, CH₂-OMMTr); 3,77 (s, 3H, O-CH₃); 3,79 (s, 3H, O-CH₃); 3,96-4,20 (m, 3H, O-CH₂-CH); 6,76-6,90 (m, 4H, Ar-H); 7,15-7,55 (m, 14H, Ar-H);
MS (FAB+LiCl): m/e = 477.2 (M+Li⁺, 20%); 470.2 (M⁺, 10%)

### 3d) (4-Methoxyphenyl)-3-O-(4-methoxytriphenylmethyl)-2-O-succinylpropylether

Die Synthese erfolgte analog Beispiel 1e aus (4-Methoxyphenyl)-3-O-(4-methoxytriphenylmethyl)-2-hydroxypropylether (Beispiel 3c).
Ausbeute: 98%;
¹H-NMR (200MHz, CDCl₃/TMS): d = 2,63 (s, 4H, CO-(CH₂)₂-CO); 3,31-3,40 (m, 2H, CH₂-OMMTr); 3,76 (s, 3H, O-CH₃); 3,79 (s, 3H, O-CH₃); 4,04-4,10 (m, 2H, CH₂-O-MOP); 5,35 (dt, 1H, CH-Osucc); 6,79 (s, 4H, Ar-H); 7,15-7,47 (m, 14H, Ar-H);
MS (FAB+LiCl): m/e = 583.3 (M+2Li⁺-H⁺, 40%); 577.3 (M+Li⁺, 100%);

### 4) Synthese von Lävulinsäure-(6-O-(4-methoxytriphenylmethyl)-5-O-succinyl)hexylester

### 4a) Lävulinsäure-4-(2,2-dimethyl-dioxolan-4-yl)butylester

0.81g (5 mmol) 2,2-Dimethyl-4-hydroxybutyl-1,3-dioxolan aus 1 a wurden zweimal mit absol. Acetonitril koevaporiert, dann zusammen mit 1,5g (7mmol) Lävulinsäureanhydrid und 0.86g (7mmol) Dimethylaminopyridin (DMAP) in absol. Pyridin gelöst und 15 h bei Raumtemperatur gerührt. Das Lösungsmittel wurde im Vakuum eingedampft und es wurde noch dreimal mit Toluol koevaporiert. Der Rückstand wurde in EE aufgenommen, die organische Phase mit gesättigter NaCl-Lösung und mit Wasser gewaschen, dann über Natriumsulfat getrocknet. Das Lösungsmittel wurde abgedampft, der Rückstand mit EE über Kieselgel chromatographiert.
Ausbeute: 0,65g (48%);
¹H-NMR (200MHz, CDCl₃/TMS): d = 1,37 (s, 3H, CH₃); 1,41 (s, 3H, CH₃); 1,42-1,75 (m, 6H, -(CH₂)₃-); 2,19 (s, 3H, CH₃-CO); 2,49-2,82 (m, 4H, COCH₂CH₂CO); 3,45-3,58 (m, 1H, C^{4'}-H); 3,97-4,16 (m, 4H, -C^{5'}H₂- & CH₂-OCO);
MS (El): m/e = 273 (M+H⁺, 45%); 257 (35%)

### 4b) Lävulinsäure-5,6-dihydroxyhexylester

Die Synthese erfolgte analog zu Beispiel 1c aus Lävulinsäure-(4-(2,2-dimethyl-dioxolan-4-yl)butylester (Beispiel 4a).
Ausbeute: 90%;
¹H-NMR (200MHz, CDCl₃/TMS): d =1,37-1,75 (m, 6H, -(CH₂)₃-); 2,20 (s, 3H, CH₃-CO); 2,47- 2,82 (m, 4H, COCH₂CH₂CO); 3,39-3,52 (dd, 1H, CH-OH); 3,60-3,79 (m, 2H, CH₂-OH); 4,11 (t, 2H, CH₂-OLaev);
MS (El): m/e = 233 (M+H⁺, 20%); 215 (15%)

### 4c) Lävulinsäure-6-O-(4-methoxytriphenylmethyl)-5-hydroxyhexylester

Die Synthese erfolgte analog zu Beispiel 1d aus Lävulinsäure-5,6-dihydroxyhexylester (Beispiel 4b).
Ausbeute: 40%;
¹H-NMR (200MHz, CDCl₃/TMS): d =1,22-1,70 (m, 6H, -(CH₂)₃-); 2,19 (s, 3H, CH₃-CO); 2,48-2,79 (m, 4H, COCH₂CH₂CO); 2,97-3,21 (m, 2H, CH₂-OMMTr); 3,79 (s, 3H, OCH₃); 3,68-3,82 (m, 1H, CH-OH); 4,03 (t, 2H, CH₂-OLaev); 6,80-7,48 (m, Ar-H, 14H)
MS (ES⁺ + LiCl): m/e = 511 (M+Li⁺, 100%)

### 4d) Lävulinsäure-(6-O-(4-methoxytriphenylmethyl)-5-O-succinyl)hexylester

Die Synthese erfolgte analog zu Beispiel 1e aus Lävulinsäure-1-(6-O-(4-methoxytriphenylmethyl)-5-hydroxy)hexylester (Beispiel 4c).
Ausbeute: 80%;
¹H-NMR (200MHz, CDCl₃/TMS): d = 1,20-1,72 (m, 6H, -(CH₂)₃-); 2,19 (s, 3H, CH₃-CO); 2,49-2,80 (m, 8H, 2 x COCH₂CH₂CO); 3,15 (d, 2H, CH₂-OMMTr); 3,79 (s, 3H, OCH₃); 4,03 (t, 2H, CH₂-OLaev); 5,15 (m, 1H, CH-OSucc); 6,79-7,50 (m, Ar-H, 14H)
MS (ES⁺ + LiCl): m/e = 627 (M+Na⁺, 20%); 611 (M+Li⁺, 50%)

### 5) Herstellung eines Trägers der Formel VIII-1 durch Beladung von Aminopropyl-CPG mit (4-Methoxyphenyl)-6-O-(4-methoxytriphenylmethyl)-5-O-succinylhexylether

123 mg (20 mmol)(4-Methoxyphenyl)-1-(6-O-(4-methoxytriphenylmethyl)-5- O-succinyl)hexylether (aus Beispiel 1) wurden zweimal mit absol. Acetonitril koevaporiert und anschließend zusammen mit 7,1 mg (22 mmol) O-(Benzotriazol-1-yl)-N,N,N',N'-tetramethyluroniumtetrafluoroborat (TBTU) und 3,2 mg (28 mmol) N-Ethylmorpholin in 0,75 ml absol. Dimethylformamid (DMF) gelöst. Zu dieser Lösung wurden 100 mg Aminopropyl-CPG (0.1 mmol/g, 550A) der Firma Fluka zugegeben und die Suspension wurde 7 h bei Raumtemperatur geschüttelt. Der derivatisierte Träger wurde abgesaugt, mit Methanol, DMF, THF, Acetonitril, wieder mit Methanol und mit Methylenchlorid gewaschen und 1h bei 40 °C im Vakuum getrocknet. Die Beladung des Trägers mit Monomethoxytritylhaltiger Komponente betrug 12,2 mmol/g. Die Verkappung reaktiver Gruppen wird am DNA Synthesizer mit Verkappungsreagenz (Acetanhydrid/2,6-Lutidin/1-Methylimidazol; je 0.25M in THF) vorgenommen, anschließend mit Acetonitril gespült.

### 6) Herstellung eines Trägers der Formel VIII-2 durch Beladung von Aminopropyl-CPG mit (4-Methoxyphenyl)-(2,2-dimethyl-dioxolan-4-yl)methylether

Herstellung analog Beispiel 5 unter Verwendung von (4-Methoxyphenyl)-(2,2-dimethyl-dioxolan-4-yl)methylether (aus Beispiel 3). Die Beladung des Trägers mit Monomethoxytritylhaltiger Komponente betrug 36,7 mmol/g.

### 7) Herstellung eines Trägers der Formel VIII-3 durch Beladung von Aminopropyl-CPG mit Lävulinsäure-(6-O-(4-methoxytriphenylmethyl)-5-O-succinyl)hexylester

Herstellung analog Beispiel 5 unter Verwendung von Lävulinsäure-(6-O-(4-methoxytriphenylmethyl)-5-O-succinyl)hexylester (aus Beispiel 4). Die Beladung des Trägers mit Monomethoxytritylhaltiger Komponente betrug 14,3 mmol/g.

### 8) Herstellung eines Trägers der Formel VIII-4 durch Beladung von ®Tentagel mit (4-Methoxyphenyl)-6-O-(4-methoxytriphenylmethyl)-5-O-succinylhexylether

306 mg (0,5 mmol) (4-Methoxyphenyl)-6-O-(4-methoxytriphenylmethyl)-5-O-succinylhexylether (aus Beispiel 1) wurden zweimal mit absol. Acetonitril koevaporiert und in einer Mischung aus 1,25 ml absol. THF und 65 ml absol. Pyridin gelöst. Dann wurde eine Lösung von 70 mg (0,5 mmol) 4-Nitrophenol und 115 mg (0,55 mmol) Dicyclohexylcarbodiimid (DCC) in 0,35 ml absol. THF zugegeben und es wurde 2h bei Raumtemperatur gerührt. Nach vollständiger Umsetzung wurde vom ausgefallenen Dicyclohexylharnstoff abzentrifugiert. Das Sediment wurde nochmals in 1 ml Ether aufgeschlämmt und abermals zentrifugiert. 200 mg ®Tentagel-Harz (PS/POE-Copolymer mit 175 mmol/g Aminofunktion) wurden in einer Mischung von 0,7 ml absol. DMF und 0,14 ml TEA aufgeschlämmt und mit der oben gewonnenen Lösung des Succinat-4-Nitrophenylesters versetzt und 17 h bei Raumtemperatur geschüttelt. Es wurde abgesaugt und wie in Beispiel 5 beschrieben aufgearbeitet. Die Beladung des Trägers mit Monomethoxytritylhaltiger Komponente betrug 28,7 mmol/g.

Oligonucleotidsynthese: Die Oligonucleotide werden zunächst durch Butanol-Fällung (Sawadogo, Van Dyke, Nucl. Acids Res. 19 (1991) 674) gereinigt. Anschließend wird das Natriumsalz erhalten durch Ausfällung aus einer 0.5 M NaCl Lösung mit 2.5 Volumenteilen Ethanol.

Die Analyse der Oligonucleotide erfolgt durch
a) Analytische Gelelektrophorese in 20% Acrylamid, 8M Harnstoff, 454M Tris-borat Puffer, pH 7.0 und/oder
b) HPLC-Analyse: Waters GenPak FAX, Gradient CH₃CN (400ml), H₂O (1.6l), NaH₂PO₄ (3.1g), NaCl (11.7g), pH6.8 (0.1M NaCl) nach CH₃CN (400ml), H₂O (1.6l), NaH₂PO₄ (3.1g), NaCl (175.3g), pH6.8 (1.5M NaCl) und/oder
c) Kapillargelelektrophorese Beckmann Kapillare eCAP™, U100P Gel Column, 65 cm length, 100 mm I.D., window 15 cm from one end, Puffer 140 µM Tris, 360mM Borsäure, 7M Harnstoff und/oder
d) Elektrospray Massenspektroskopie

### 9) Herstellung von Oligonucleotiden der Formel I: TpTpTpTpTpTpTpTp-CH₂₋CH(OH)(CH₂)₄-(O-Methoxyphenyl)

Das Monomer ist jeweils ein β-D-Desoxyribonucleosid; R¹=R²=H; Z = O-(4-Methoxyphenyl); n=8, m=m'=n'=b=0; A=V=W=U=X=Y=T= Oxy; a=3;
a) 0.2 µmol des Trägers VIII-4 aus Beispiel 8 werden nacheinander mit folgenden Reagenzien behandelt:
   1. Acetonitril abs.
   2. 3 % Trichloressigsäure in Dichlormethan
   3. Acetonitril abs.
   4. 4 µmol 5'-O-Dimethoxytritylthymidin-3'-phosphorigsäure-β-cyanoethylester-diisopropylamid und 25 µmol Tetrazol in 0.15 ml Acetonitril abs.
   5. Acetonitril
   6. 20 % Acetanhydrid in THF mit 40% Lutidin und 10 % Dimethylaminopyridin
   7. Acetonitril
   8. Jod (0.1 M J₂ in THF/Wasser/Pyridin; 70:20:5 =v:v:v)

   Die Schritte 1 bis 8, nachfolgend ein Reaktionszyklus genannt, werden zum Aufbau des Octathymidylat-Derivats 7-mal wiederholt.
b) Nach abgeschlossener Synthese erfolgt die Abspaltung der Dimethoxytritylgruppe wie in den Schritten 1 bis 3 beschrieben.
c) Durch 1.5-stündige Behandlung mit Ammoniak wird das Oligonucleotid vom Träger gespalten und zugleich werden die β-Cyanoethyl-Gruppen eliminiert. Da das Oligonucleotid keine Amino-Schutzgruppen enthält, ist keine weitere Ammoniak-Behandlung notwendig.

### 10) Herstellung von Oligonucleotiden der Formel I: TpTpTpTpTpTpTpTp-CH₂₋CH(OH)(CH₂)₄-(OH)

Das Monomer ist jeweils ein β-D-Desoxyribonucleosid; R¹=R²=H; Z = OH; n=8, m=m'=n'=b=0; A=V=W=U=X=Y=T= Oxy; a=3;
a) Die Herstellung erfolgt analog Beispiel 9a;
b) Nach abgeschlossener Synthese erfolgt die Abspaltung der Dimethoxytritylgruppe (DMTr-Gruppe) wie in den Schritten 1 bis 3 beschrieben. Anschließend wird die 4-Methoxyphenylgruppe (MOP-Gruppe) durch eine Behandlung mit einer 0, 1 M Ce^{IV}(NH₄)₂(NO₃)₆ in Acetonitril/H₂O 4:1 bei Raumtemperatur für 5 min abgespalten.
c) Durch 1.5-stündige Behandlung mit Ammoniak wird das Oligonucleotid vom Träger gespalten und zugleich werden die β-Cyanoethyl-Gruppen eliminiert.

### 11) Herstellung von Oligonucleotiden der Formel I: TpTpTpTpTpTpTpTp-CH₂₋CH(OH)(CH₂)₄-(O-(CH₂)₄-Pyren) ausgehend von Träger VIII-4

Das Monomer ist jeweils ein β-D-Desoxyribonucleosid; R¹=R²=H; Z = O-(-(CH₂)₄-Pyren); n=8, m=m'=n'=b=0; A=V=W=U=X=Y=T= Oxy; a=3;
a) Die Herstellung erfolgt analog Beispiel 9a;
b) Nach abgeschlossener Synthese erfolgt die Abspaltung der Dimethoxytritylgruppe wie in den Schritten 1 bis 3 beschrieben. Nachfolgend wird die entstandene freie 5'-Hydroxygruppe wie in den Schritten 6 und 7 beschrieben gecappt. Anschließend wird die 4-Methoxyphenylgruppe durch eine Behandlung mit einer 0,1M Ce^{IV}(NH₄)₂(NO₃)₆ in Acetonitril/H₂O 4:1 bei Raumtemperatur für 5 min abgespalten.
c) Die Einführung des [4-(1-Pyrenyl)butyl]phosphodiesters am 5'-Ende erfolgt wie in J. S. Mann et al. Bioconj. Chem. 3 (1992) 554 beschrieben durch Behandlung mit 4 µmol 4-(1-Pyrenyl)butyl-2-cyanoethyl-N,N-Diisopropylphosphoramidit und 25 *µ*mol Methylthio-1H-tetrazol in 0.15 ml Acetonitril abs. und anschließendem Waschen mit Acetonitril.
d) Oxidation mit 0.1M J₂ in THF/Wasser/Pyridin; 70:20:5 = v:v:v.
e) Durch 1.5-stündige Behandlung mit Ammoniak wird das Oligonucleotid vom Träger gespalten und zugleich werden die β-Cyanoethyl-Gruppen eliminiert.

### 12) Herstellung von Oligonucleotiden der Formel I: TpTpTpTpTpTpTpTp-CH₂₋CH(OH)(CH₂)₄(O-(CH₂)₄-Pyren) ausgehend von Träger VIII-1

Das Monomer ist jeweils ein β-D-Desoxyribonucleosid; R¹ = R² = H; Z = O-(-(CH₂)₄-Pyren); n=8, m=m'=n'=b=0; A=V=W=U=X=Y=T= Oxy; a=3;
Die Herstellung erfolgt analog Beispiel 9a, jedoch unter Verwendung von Träger VIII-1.

### 13) Herstellung von Oligonucleotiden der Formel I: TpTpTpTpTpTpTpTp-CH₂₋CH(OH)(CH₂)₄-(O-(CH₂)₁₁CH₃) ausgehend von Träger VIII-4

Das Monomer ist jeweils ein β-D-Desoxyribonucleosid; R¹= R²= H; Z = O-(CH₂)₁₁CH₃; n=8, m=m'=n'=b=0; A=V=W=U=X=Y=T= Oxy; a=3;
a) Die Herstellung erfolgt analog Beispiel 9a;
b) Abspaltung der DMTr-Gruppe, Capping und Abspaltung der MOP-Gruppe wie in Beispiel 11b beschrieben;
c) Behandlung mit 4 µmol Dodecyl-2-cyanoethyl-N,N-Diisopropylphosphoramidit und 25 µmol Methylthio-1H-tetrazol in 0.15 ml Acetonitril abs. und anschließendem Waschen mit Acetonitril.
d) Oxidation mit 0.1M J₂ in THF/Wasser/Pyridin; 70:20:5 = v:v:v.
e) Durch 1.5-stündige Behandlung mit Ammoniak wird das Oligonucleotid vom Träger gespalten und zugleich werden die β-Cyanoethyl-Gruppen eliminiert.

### 14) Herstellung von Oligonucleotiden der Formel I: TpTpTpTpTpTpTpTp-CH₂₋CH(OH)(CH₂)₄-(O-(CH₂)₁₃CH₃) ausgehend von Träger VIII-1

Das Monomer ist jeweils ein β-D-Desoxyribonucleosid; R¹= R²= H; Z = O-(CH₂)₁₃CH₃; n=8, m=m'=n'=b=0; A=V=W=U=X=Y=T= Oxy; a=3;
a) Die Herstellung erfolgt analog Beispiel 9a;
b) Abspaltung der DMTr-Gruppe, Capping und Abspaltung der MOP-Gruppe wie in Beispiel 11b beschrieben;
c) Behandlung mit 4 *µ*mol Tetradecyl-2-cyanoethyl-N,N-Diisopropylphosphoramidit und 25 µmol Methylthio-1H-tetrazol in 0.15 ml Acetonitril abs. und anschließendem Waschen mit Acetonitril.
d) Oxidation mit 0.1M J₂ in THF/Wasser/Pyridin; 70:20:5 = v:v:v.
e) Durch 1.5-stündige Behandlung mit Ammoniak wird das Oligonucleotid vom Träger gespalten und zugleich werden die β-Cyanoethyl-Gruppen eliminiert.

### 15) Herstellung von Oligonucleotiden der Formel I: TpTpTpTpTpTpTpTp-CH₂-CH(OH)(CH₂)-(O-3'-T-ODMtr) ausgehend von Träger VIII-2

Das Monomer ist jeweils ein β-D-Desoxyribonucleosid; R¹=R²=H; Z = O-3'-T-ODMTr; n=8, m=m'=n'=b=0; A=V=W=U=X=Y=T= Oxy; a=0;
a) Die Herstellung erfolgt analog Beispiel 9a;
b) Abspaltung der DMTr-Gruppe, Capping und Abspaltung der MOP-Gruppe wie in Beispiel 11b beschrieben;
c) Behandlung mit 4 µmol 5'-O-Dimethoxytritylthymidin-3'-phosphorigsäure-β-cyanoethylester-diisopropylamid und 25 µmol Methylthio-1H-tetrazol in 0.15 ml Acetonitril abs. und anschließendem Waschen mit Acetonitril.
d) Oxidation mit 0.1M J₂ in THF/Wasser/Pyridin; 70:20:5 = v:v:v.
e) Durch 1.5-stündige Behandlung mit Ammoniak wird das Oligonucleotid vom Träger gespalten und zugleich werden die β-Cyanoethyl-Gruppen eliminiert.

### 16) Herstellung von Oligonucleotiden der Formel I: TpTpTpTpTpTpTpTp-(CH₂)₄-CH(OH)-CH₂-(O-(CH₂)₁₃CH₃) ausgehend von Träger VIII-4

Das Monomer ist jeweils ein β-D-Desoxyribonucleosid; R¹= R²= H; Z = O-(4-Methoxyphenyl); n=8, m=m'=n'=a=0; A=V=W=U=X=Y=T= Oxy; b=3;
a) 0.2 µmol des Trägers VIII-4 aus Beispiel 8 werden nacheinander mit folgenden Reagenzien behandelt:
   1. Acetonitril abs.
   2. 3 % Trichloressigsäure in Dichlormethan
   3. Acetonitril abs.
   4. 4 *µ*mol Tetradecyl-2-cyanoethyl-N,N-Diisopropylphosphoramidit und 25 µmol Methylthio-1H-tetrazol in 0.15 ml Acetonitril abs.
   5. Acetonitril
   6. 0,1M Ce^{IV}(NH₄)₂(NO₃)₆ in Acetonitril/H₂O 4:1 bei Raumtemperatur für 5 min
   7. Acetonitril
   8. 4 *µ*mol 5'-O-Dimethoxytritylthymidin-3'-phosphorigsäure-β-cyanoethylester-diisopropylamid und 25 µmol Tetrazol in 0.15 ml Acetonitril abs.
   9. Acetonitril
   10. 20 % Acetanhydrid in THF mit 40% Lutidin und 10 % Dimethylaminopyridin
   11. Acetonitril
   12. Jod (1.3 g in THF/Wasser/Pyridin; 70:20:5=v:v:v)
   13. Acetonitril
   14. 3% Trichloressigsäure in Dichlormethan

   Die Schritte 7 - 14, nachfolgend ein Reaktionszyklus genannt, werden zum Aufbau des Octathymidylat-Derivats 7-mal wiederholt.
b) Durch 12-stündige Behandlung mit Ammoniak bei 60 °C wird das Oligonucleotid vom Träger gespalten und zugleich werden die β-Cyanoethyl-Gruppen eliminiert.

### 17) Herstellung von Oligonucleotiden der Formel I: GₚGₚAₚCₚCₚGₚAₚAₚGₚGₚ-(CH₂)₄-CH(OH)-CH₂-(O-(CH₂)₁₃CH₃) ausgehend von Träger VIII-4

Das Monomer ist jeweils ein β-D-Desoxyribonucleosid; R¹= R²= H; Z = O-(4-Methoxyphenyl); n=10, m=m'=n'=a=0; A=V=W=U=X=Y=T= Oxy; b=3;
Die Synthese erfolgt analog Beispiel 16, jedoch wird in Schritt 8 das jeweilige 3'-Phosphorigsäure-β-cyanoethylester-diisopropylamid der entsprechenden Base eingesetzt.

### 18) Herstellung von Oligonucleotiden der Formel I: TpTpTpTpTpTpTpTp-(CH₂)₄-CH(OH)-CH₂-(O-(CH₂)₁₃CH₃) ausgehend von Träger VIII-3

Das Monomer ist jeweils ein β-D-Desoxyribonucleosid; R¹=R²=H; Z = O-(4-Methoxyphenyl); n=8, m=m'=n'=a=0; A=V=W=U=X=Y=T= Oxy; b=3;
Die Synthese erfolgt analog Beispiel 16, jedoch wird Schritt 6 durch Behandlung mit 0,5M Hydrazinhydrat in Essigsäure/Pyridin 2:3 während 30 min ersetzt.

### 19) Herstellung von Oligonucleotiden der Formel I: TpTpTpTpTpTpTpTp-CH₂₋CH(OH) (CH₂)₄-(O-Acridin) ausgehend von Träger VIII-4

Das Monomer ist jeweils ein β-D-Desoxyribonucleosid; R¹ = R² = H; Z = Acridin, wobei Acridin steht für 6-(2-Methoxy-6-chloro-9-acridin)amino)-2-hydroxymethylhexoxy; n=8, m=m'=n'=b=0; A=V=W=U=X=Y=T= Oxy; a =3;
a) Die Herstellung erfolgt analog Beispiel 9a;
b) Abspaltung der DMTr-Gruppe, Capping und Abspaltung der MOP-Gruppe wie in 11b beschrieben;
c) Behandlung mit 4 µmol 6-(2-Methoxy-6-chloro-9-acridin)amino)-2-dimethoxytrityloxymethyl-1-(2-cyanoethyl-N,N-diisopropylphosphino)hexan (Fa. Glen Research) und 25 µmol Methylthio-1H-tetrazol in 0.15 ml Acetonitril abs. und anschließendem Waschen mit Acetonitril.
d) Oxidation mit 0.1M J₂ in THF/Wasser/Pyridin; 70:20:5 =v:v:v und Waschen mit Acetonitril
e) Abspaltung der DMTr-Gruppe
f) Durch 1.5-stündige Behandlung mit Ammoniak wird das Oligonucleotid vom Träger gespalten und zugleich werden die β-Cyanoethyl-Gruppen eliminiert.

### 20) Herstellung von Oligonucleotiden der Formel I: TpTpTpTpTpTpTpTp-CH₂₋CH(OH) (CH₂)₄-(O-Biotin) ausgehend von Träger VIII-4

Das Monomer ist jeweils ein β-D-Desoxyribonucleosid; R¹ = R² = H; Z = Biotin, wobei Biotin steht für 6-Biotinamido-5-hydroxymethyl-hexoxy;
n=8, m=m'=n'=b=0; A=V=W=U=X=Y=T= Oxy; a=3;
a) Die Herstellung erfolgt analog Beispiel 9a;
b) Abspaltung der DMTr-Gruppe, Capping und Abspaltung der MOP-Gruppe wie in 11b beschrieben;
c) Behandlung mit 4 µmol 6-Biotinamido-5- dimethoxytrityloxymethyl-hexan-(2-cyanoethyl-N,N-diisopropylphosphoramidit) (Fa. Glen Research) und 25 mol Methylthio-1H-tetrazol in 0.15 ml Acetonitril abs. und anschließendem Waschen mit Acetonitril.
d) Oxidation mit 0.1M J₂ in THF/Wasser/Pyridin; 70:20:5 =v:v:v und Waschen mit Acetonitril
e) Abspaltung der DMTr-Gruppe
f) Durch 1.5-stündige Behandlung mit Ammoniak wird das Oligonucleotid vom Träger gespalten und zugleich werden die β-Cyanoethyl-Gruppen eliminiert.

### 21) Herstellung von Oligonucleotiden der Formel I: TpTpTpTpTpTpTpTp-CH₂₋CH(OH) (CH₂)₄-(O-TEGBiotin) ausgehend von Träger VIII-4

Das Monomer ist jeweils ein β-D-Desoxyribonucleosid; R¹=R²=H; Z = TEGBiotin, wobei TEGBiotin steht für 16-Biotinamido-4,7,10,13-tetraoxy-1-hydroxy-hexadecan-2-oxy; n=8, m=m'=n'=b=0; A=V=W=U=X=Y=T= Oxy; a=3;
a) Die Herstellung erfolgt analog Beispiel 9a;
b) Abspaltung der DMTr-Gruppe, Capping und Abspaltung der MOP-Gruppe wie in 11b beschrieben;
c) Behandlung mit 4 µmol 16-Biotinamido-4,7,10,13-tetraoxy-1-dimethoxytrityloxy-2-hexadecan-(2-cyanoethyl-N,N-diisopropylphosphoramidit) (Fa. Glen Research) und 25 µmol Methylthio-1H-tetrazol in 0.15 ml Acetonitril abs. und anschließendem Waschen mit Acetonitril.
d) Oxidation mit 0.1M J₂ in THF/Wasser/Pyridin; 70:20:5 =v:v:v und Waschen mit Acetonitril
e) Abspaltung der DMTr-Gruppe
f) Durch 1.5-stündige Behandlung mit Ammoniak wird das Oligonucleotid vom Träger gespalten und zugleich werden die β-Cyanoethyl-Gruppen eliminiert.

### 22) Herstellung von Oligonucleotiden der Formel I: TpTpTpTpTpTpTpTp-CH₂₋CH(OH) (CH₂)₄-(O-Cholesterin) ausgehend von Träger VIII-4

Das Monomer ist jeweils ein β-D-Desoxyribonucleosid; R¹= R² = H; Z =Cholesterin, wobei Cholesterin steht für 16-N-Cholesteryaminol-4,7,10,13-tetraoxy-1-hydroxy-hexadecan-2-oxy; n=8, m=m'=n'=b=0;
A=V=W=U=X=Y=T= Oxy; a=3;
a) Die Herstellung erfolgt analog Beispiel 9a;
b) Abspaltung der DMTr-Gruppe, Capping und Abspaltung der MOP-Gruppe wie in 11b beschrieben;
c) Behandlung mit 4 µmol 16-N-Cholesterylamino-4,7,10,13-tetraoxy-1-dimethoxytrityloxy-2-hexadecan-(2-cyanoethyl-N,N-diisopropylphosphoramidit) (Fa. Glen Research) und 25 µmol Methylthio-1H-tetrazol in 0.15 ml Acetonitril abs. und anschließendem Waschen mit Acetonitril.
d) Oxidation mit 0.1M J₂ in THF/Wasser/Pyridin; 70:20:5 =v:v:v und Waschen mit Acetonitril
e) Abspaltung der DMTr-Gruppe
f) Durch 1.5-stündige Behandlung mit Ammoniak wird das Oligonucleotid vom Träger gespalten und zugleich werden die β-Cyanoethyl-Gruppen eliminiert.

### 23) Herstellung von Oligonucleotiden der Formel I: TpTpTpTpTpTpTpTp-CH₂₋CH(OH) (CH₂)₄-(O-Psoralen) ausgehend von Träger VIII-4

Das Monomer ist jeweils ein β-D-Desoxyribonucleosid; R¹ = R² = H; Z = Psoralen, wobei Psoralen steht für 2-[4'-(Hydroxymethyl)-4,5',8-trimethylpsoralen]-ethyl-; n=8, m=m'=n'=b=0; A=V=W=U=X=Y=T= Oxy; a=3;
a) Die Herstellung erfolgt analog Beispiel 9a;
b) Abspaltung der DMTr-Gruppe, Capping und Abspaltung der MOP-Gruppe wie in 11b beschrieben;
c) Behandlung mit 4 µmol 2-[4'-(Hydroxymethyl)-4,5',8-trimethylpsoralen]-ethyl-2-cyanoethyl-N,N-diisopropylphosphoramidit (Fa. Glen Research) und 25 µmol Methylthio-1H-tetrazol in 0.15 ml Acetonitril abs. und anschließendem Waschen mit Acetonitril.
d) Oxidation mit 0.1M J₂ in THF/Wasser/Pyridin; 70:20:5 =v:v:v und Waschen mit Acetonitril
e) Durch 1.5-stündige Behandlung mit Ammoniak wird das Oligonucleotid vom Träger gespalten und zugleich werden die β-Cyanoethyl-Gruppen eliminiert.

### 24) Herstellung von Oligonucleotiden der Formel I: TpTpTpTpTpTpTpTp-CH₂₋CH(OH)(CH₂)-(O-(CH₂)₁₃CH₃) ausgehend von Träger VIII-2

Das Monomer ist jeweils ein β-D-Desoxyribonucleosid; R¹ = R² = H; Z = O-(CH₂)₁₃CH₃; n=8, m=m'=n'=b=0; A=V=W=U=X=Y=T= Oxy; a=0;
a) 0.2 *µ*mol des Trägers VIII-2 aus Beispiel 6 werden nacheinander behandelt mit:
   1. Acetonitril abs.
   2. 3 % Trichloressigsäure in Dichlormethan
   3. Acetonitril abs.
   4. 4 *µ*mol 1-Tetradecyl-[(2-cyanoethyl)-N,N-diisopropylphosphoramidit] und 25 *µ*mol Methylthio-1H-tetrazol in 0.15 ml Acetonitril abs.
   5. Acetonitril
   6. 20 % Acetanhydrid in THF mit 40% Lutidin und 10 % Dimethylaminopyridin
   7. Acetonitril
   8. Jod (0.1 M J₂ in THF/Wasser/Pyridin; 70:20:5 =v:v:v)
   9. 0.1M Ce^{IV}(NH₄)₂(NO₃)₆ in Acetonitril/H₂O 4:1 (s. auch Beispiel 11b).
   10. Acetonitril
b) und anschließend behandelt mit
   1. 4 µmol 5'-O-Dimethoxytritylthymidin-3'-phosphorigsäure-β-cyanoethylester-diisopropylamid und 25 µmol Tetrazol in 0.15 ml Acetonitril abs.
   2. Acetonitril
   3. 20 % Acetanhydrid in THF mit 40% Lutidin und 10 % Dimethylaminopyridin
   4. Acetonitril
   5. Jod (0.1M J₂ in THF/Wasser/Pyridin; 70:20:5 =v:v:v)
   6. Acetonitril abs.
   7. 3 % Trichloressigsäure in Dichlormethan
   8. Acetonitril abs.

   Die Schritte 1 bis 8 , nachfolgend ein Reaktionszyklus genannt, werden zum Aufbau des Octathymidylat-Derivats 7-mal wiederholt.
c) Durch 1.5-stündige Behandlung mit Ammoniak wird das Oligonucleotid vom Träger gespalten und zugleich werden die β-Cyanoethyl-Gruppen eliminiert. Da das Oligonucleotid keine Amino-Schutzgruppen enthält, ist keine weitere Ammoniak-Behandlung notwendig.

### 25) Herstellung von Oligonucleotiden der Formel I: CpApCpGpTpTpGpApGpGpGpGpCpApTp-CH₂₋CH(OH)(CH₂)-(O-(CH₂)₁₃CH₃) ausgehend von Träger VIII-2

Das Monomer ist jeweils ein β-D-Desoxyribonucleosid; R¹ = R² = H; Z = O-(CH₂)₁₃CH₃; n=15, m=m'=n'=b=0; A=V=W=U=X=Y=T= Oxy; a=0;
Synthese analog Beispiel 24, jedoch unter Verwendung der entsprechenden Standard 5'-O-Dimethoxytritylthymidin geschützten 3'-(2-Cyanoethyl)-N,N-diisopropylphosphoramidit-Nucleoside in Schritt b1. Durch 1.5-stündige Behandlung mit Ammoniak wird das Oligonucleotid vom Träger gespalten, die Entschützung erfolgte durch 16h Behandlung mit Ammoniak bei 60°C.

### 26) Herstellung von Oligonucleotiden der Formel I: CpApCpGpTpTpGpApGpGpGpGpCpApTp-CH₂₋CH(OH)(CH₂)-(O-Vitamin E) ausgehend von Träger VIII-2

Das Monomer ist jeweils ein β-D-Desoxyribonucleosid; R¹ = R² = H; Z = O-Vitamin E; n=15, m=m'=n'=b=0; A=V=W=U=X=Y=T = Oxy; a=0;
Synthese analog Beispiel 24, jedoch unter Verwendung von Vitamin E-(2-Cyanoethyl)-N,N-diisopropylphosphoramidit in Schritt a4.

### 27) Synthese von Laevulinsäure-(3-O-(4-methoxytriphenylmethyl)-2-O-succinyl)propylester

### 27a) Laevulinsäure- (2,2-dimethyl-dioxolan-4-yl)methylester

Synthese analog Beispiel 4a aus 2,2-Dimethyl-4-hydroxymethyl-1,3-dioxolan.
Ausbeute: 71%.
¹H-NMR (200MHz, CDCl₃/TMS): δ = 1,38 (s, 3H, CH₃); 1,42 (s, 3H, CH₃); 2,19 (s, 3H, CH₃-CO); 2,51-2,82 (m, 4H, COCH₂CH₂CO); 3,75 (dd, 1H, C^{4'}-H); 4.01-4.39 (m, 4H, -C^{5'}H₂- & CH₂-OCO);

### 27b) Laevulinsäure-1-(2,3-dihydroxy)propylester

Synthese analog zu 1c aus Laevulinsäure-(2,2-dimethyl-dioxolan-4-yl)methylester (27a);
Ausbeute: 90%;
¹H-NMR (200MHz, CDCl₃/TMS): δ = 2,20 (s, 3H, CH₃-CO); 2,60, 2,80 (jew. t, 4H, COCH₂CH₂CO); 3.54-3.80 (m, 2H, CH₂-OH); 3.80 (t,1H, OH); 3.95 (m, 1H, CH-OH); 4,21 (d, 2H, CH₂-OLaev);

### 27c) Lävulinsäure-3-O-(4-methoxytriphenylmethyl)-2-hydroxypropylester

Synthese analog zu 1d aus Lävulinsäure-(2,3-dihydroxy)propylester (24b);
Ausbeute: 20%;

### 27d) Lävulinsäure-3-O-(4-methoxytriphenylmethyl)2-O-succinylpropylester

Synthese analog zu 1e aus Lävulinsäure-3-O-(4-methoxytriphenylmethyl)-2-hydroxypropylester (27c);
Ausbeute: 51%;
MS (FAB/LiCl): m/e = 599.3 (M+Li⁺);

### 28) Herstellung eines Trägers der Formel VIII-5 durch Beladung von Aminopropyl-CPG mit Lävulinsäure-1-(3-O-(4-methoxytriphenylmethyl)-2-O-succinyl)propylester

Herstellung analog Beispiel 5 unter Verwendung von Lävulinsäure-1-(3-O-(4-methoxytriphenylmethyl)-2-O-succinyl)propylester (aus Beispiel 27). Die Beladung des Trägers mit Monomethoxytritylhaltiger Komponente betrug 24,7 *µ*mol/g.

## Patentansprüche

1. Verbindungen der Formel I 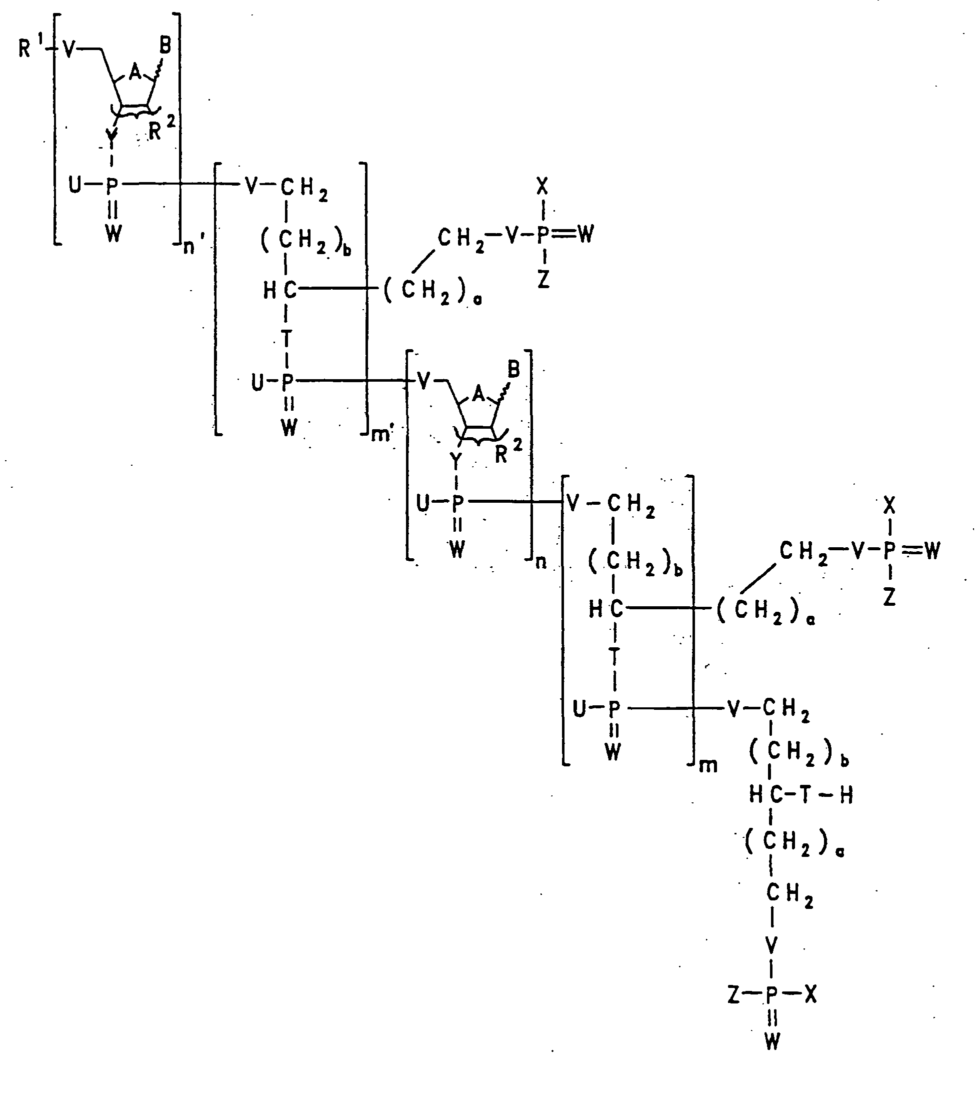 sowie deren physiologisch verträglichen Salze, worin
a eine Zahl von Null bis 20 bedeutet;
b eine Zahl von Null bis 20 bedeutet;
R¹ Wasserstoff, C₁-C₁₈-Alkyl, C₂-C₁₈-Alkenyl, C₃-C₁₈-Alkenyl, C₁-C₁₈-Alkylcarbonyl, C₂-C₁₉-Alkenylcarbonyl, C₃-C₁₉-Alkinylcarbonyl, C₆-C₂₀-Aryl, C₆-C₁₄-Aryl-C₁-C₈-alkyl, oder einen Rest der Formel III 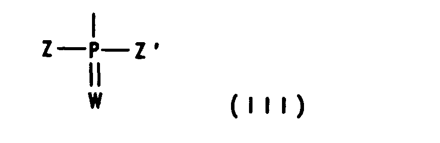 bedeutet;
R² Wasserstoff, Hydroxy, C₁-C₁₈-Alkoxy, Halogen, Azido oder NH₂ bedeutet;
D Hydroxy, O-PO₃²⁻ bedeutet;
B für eine in der Nucleotidchemie übliche Base, beispielsweise für natürliche Basen unnatürliche Basen steht;
n eine ganze Zahl von 7 bis 25 bedeutet;
n' eine ganze Zahl von Null bis 50 bedeutet;
m' in Formel I eine ganze Zahl von Null bis 5 bedeutet;
A für Oxy, Thioxy oder Methylen steht;
W Oxo, Thioxo oder Selenoxo bedeutet;
V Oxy oder Sulfandiyl bedeutet;
T Oxy, Sulfandiyl oder Imino bedeutet;
Y Oxy, Sulfandiyl, Imino oder Methylen bedeutet;
X Hydroxy oder Mercapto bedeutet;
U Hydroxy, Mercapto, BH₃, SeH, C₁-C₁₈-Alkoxy, C₁-C₁₈-Alkyl, C₆-C₂₀-Aryl, C₆-C₁₄-Aryl-C₁-C₈-alkyl, NHR³, NR³R⁴ oder einen Rest der Formel IV
(OCH₂CH₂)ₚO(CH₂)_{q}CH₂R⁵ (IV)
bedeutet, worin
R³ C₁-C₁₈-Alkyl, C₆-C₂₀-Aryl, C₆-C₁₄-Aryl-C₁-C₈-alkyl, -(CH₂)_{c}-[NH(CH₂)_{c}]_{d}-NR⁶R⁶, worin c eine ganze Zahl von 2 bis 6 und d eine ganze Zahl von Null bis 6 ist, und R⁶ unabhängig voneinander Wasserstoff, C₁-C₆-Alkyl oder C₁-C₄-Alkoxy-C₁-C₆-alkyl bedeutet;
R⁴ C₁-C₁₈-Alkyl, C₆-C₂₀-Aryl oder C₆-C₁₀-Aryl-C₁-C₈-alkyl bedeutet oder im Falle von NR³R⁴ zusammen mit R³ und dem sie tragenden Stickstoffatom einen 5-6-gliedrigen heterozyklischen Ring bedeutet, der zusätzlich ein weiteres Heteroatom aus der Reihe O, S, N enthalten kann;
p eine ganze Zahl von 1 bis 100 ist;
q eine ganze Zahl von Null bis 22 ist;
R⁵ Wasserstoff oder eine funktionelle Gruppe ausgewählt aus Hydroxy, Amino, NHR⁷, COOH, CONH₂, COOR⁸ oder Halogen bedeutet, worin R⁷ C₁-C₆-Alkyl und R⁸ C₁-C₄-Alkyl bedeutet;
Z, Z' unabhängig voneinander Hydroxy, Mercapto, SeH, C₁-C₂₂-Alkoxy, -O-(CH₂)_{b}-NR⁷R⁸, worin b eine ganze Zahl von 1 bis 6 ist, und R⁷ C₁-C₆-Alkyl und R⁸ C₁-C₄-Alkyl ist oder R⁷ und R⁸ zusammen mit dem sie tragenden Stickstoffatom einen 3-6 gliedrigen Ring bilden; C₁-C₁₈-Alkyl, vorzugsweise C₁-C₈-Alkyl, C₆-C₂₀-Aryl, C₆-C₁₄-Aryl-C₁-C₈-alkyl, vorzugsweise C₆-C₁₀-Aryl-C₁-C₄-alkyl, C₆-C₁₄-Aryl-C₁-C₈-alkoxy, vorzugsweise C₆-C₁₀-Aryl-C₁-C₄-alkoxy, wobei Aryl auch Heteroaryl bedeutet und Aryl gegebenenfalls durch 1, 2 oder 3 gleiche oder verschiedene Reste aus der Reihe Carboxy, Amino, Nitro, C₁-C₄-Alkylamino, C₁-C₆-Alkoxy, Hydroxy, Halogen und Cyano substituiert ist, C₁-C₁₈-Alkylmercapto, NHR³, NR³R⁴, worin R³ und R⁴ wie oben definiert sind, oder eine Gruppe bedeuten, die
a) die intrazelluläre Aufnahme begünstigt, nämlich lipophile Reste wie -O-(CH₂)ₓ-CH₃, worin x eine ganze Zahl von 6 bis18 bedeutet, -O-(CH₂)ₑ-CH=CH-(CH₂)_{f}-CH₃, worin e und f unabhängig voneinander eine ganze Zahl von 6 bis 12 bedeuten, -O-(CH₂CH₂O)₄-(CH₂)₉-CH₃, -O-(CH₂CH₂O)₈-(CH₂)₁₃-CH₃ und -0-(CH₂CH₂O)₇-(CH₂)₁₅-CH₃, Steroid-Reste wie Cholesteryl und Konjugate, die natürliche Carriersysteme ausnutzen wie Gallensäure, Folsäure, 2-(N-Alkyl-N-Alkoxy)-Aminoanthrachinon und Konjugate der Mannose und Peptide der entsprechenden Rezeptoren, die zur rezeptorvermittelten Endozytose der Oligonucleotide führen wie EGF (Epidermal Growth Factor), Bradykinin und PDGF (Platelet Derived Growth Factor oder
b) als Markierung einer DNA-Sonde dient, nämlich fluoreszierende Gruppen beispielsweise Dansyl- (=N-Dimethyl-1-aminonaphthyl-5-sulfonyl-), Fluorescein- oder Coumarin-Derivate oder chemilumineszierende Gruppen beispielsweise Acridin-Derivate, das Digoxigenin-System, die Biotin-Gruppe oder Linker-Arme mit funktionellen Gruppen, die eine nachträgliche Derivatisierung mit nachweisbaren Reporter-Gruppen gestatten, beispielsweise ein Aminoalkyl-Linker, der mit einem Acridinium-Aktivester zur Chemilumineszenz-Probe umgesetzt wird oder
c) bei der Hybridisierung des Oligonucleotidanalogons an die Target-Nucleinsäure diese unter Bindung, Quervernetzung oder Spaltung angreift, nämlich Acridin-, Psoralen-, Phenanthrolin, Naphthochinon-, Daunomycin- oder Chlorethylaminoaryl-Konjugate oder
d) ein über das 5'- oder 3'-Ende verknüpftes Nucleosid oder Oligonucleotid; und die geschweifte Klammer andeutet, daß sich R² und der benachbarte Phosphorylrest in 2'- und 3'-Stellung oder auch umgekehrt in 3'- und 2'-Stellung befinden können,
wobei jedes Nucleotid in seiner D- bzw. L-Konfiguration vorliegen kann und sich die Base B in α- bzw. β-Stellung befinden kann.

2. Verbindungen der Formel I gemäß Anspruch 1 sowie deren physiologisch verträglichen Salze, **dadurch gekennzeichnet, daß** sich die Base B in β-Stellung befindet, die Nucleotide in der D-Konfiguration vorliegen und R² sich in 2'-Stellung befindet.

3. Verbindungen der Formel I gemäß Anspruch 1 oder 2 sowie deren physiologisch verträglichen Salze, **dadurch gekennzeichnet, daß**
a eine Zahl von Null 10 bedeutet;
b eine Zahl von Null bis 10 bedeutet;
R¹ Wasserstoff oder einen Rest der Formel III bedeutet; 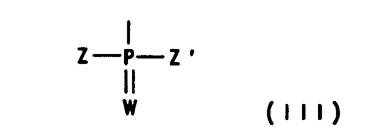
R² Wasserstoff oder Hydroxy bedeutet;
n' eine ganze Zahl von Null bis 30 bedeutet;
A für Oxy steht;
W Oxo oder Thioxo bedeutet;
U Hydroxy, Mercapto, C₁-C₆-Alkoxy, C₁-C₆-Alkyl, NR³R⁴ oder NHR³ bedeutet, worin
R³ C₁-C₈-Alkyl bedeutet; und
R⁴ C₁-C₈-Alkyl, C₆-C₂₀-Aryl oder C₆-C₁₀-Aryl-C₁-C₈-alkyl bedeutet
oder im Falle von NR³R⁴ zusammen mit R³ und dem sie tragenden Stickstoffatom einen 5-6-gliedrigen heterozyklischen Ring bedeutet, der zusätzlich ein weiteres Heteroatom aus der Reihe O, S, N enthalten kann.

4. Verbindungen der Formel I gemäß den Ansprüchen 1 bis 3 sowie deren physiologisch verträglichen Salze, **dadurch gekennzeichnet, daß**
a eine Zahl von Null bis 4 bedeutet;
b eine Zahl von Null bedeutet;
R¹ Wasserstoff bedeutet;
R² Wasserstoff bedeutet;
D Hydroxy bedeutet;
B für Adenin, Cytosin, Guanin, Uracil, Thymin, 5-Propinuracil oder 5-Propincytosin steht;
n' eine ganze Zahl von Null bis 25 bedeutet;
m Null bedeutet;
m' in Formel I eine ganze Zahl von Null oder 1 bedeutet;
T Oxy bedeutet;
Y Oxy bedeutet; und
U Hydroxy oder C₁-C₆-Alkyl bedeutet.

5. Verwendung der Verbindungen der Formel I gemäß den Ansprüchen 1 bis 4 als Sonde zum Nachweis von Nucleinsäuren in vitro.

6. Pharmazeutische Zubereitung enthaltend eine Verbindung der Formel I gemäß den Ansprüchen 1 bis 4 gegebenenfalls zusammen mit physiologisch verträglichen Hilfs- und/oder Trägerstoffen und/oder zusammen mit anderen bekannten Wirkstoffen.

## Claims

1. A compound of the formula I 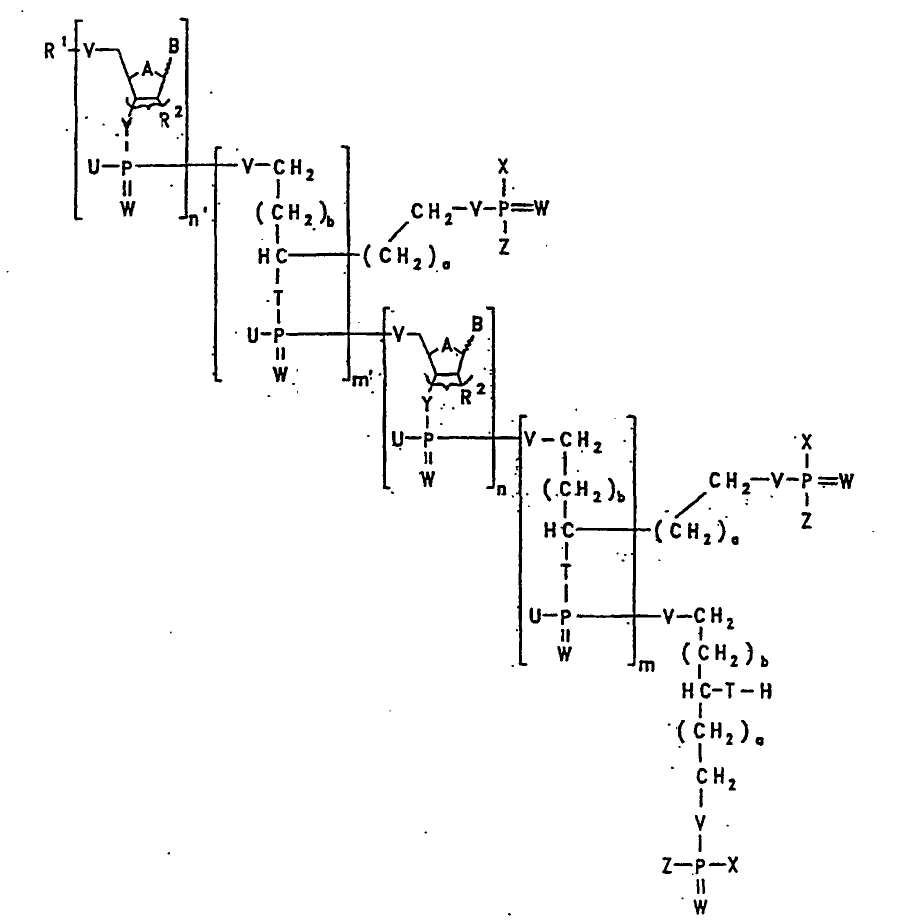 and the physiologically tolerated salts thereof, in which
a is a number from zero to 20;
b is a number from zero to 20;
R¹ is hydrogen, C₁-C₁₈-alkyl, C₂-C₁₈-alkenyl, C₃-C₁₈-alkynyl, C₁-C₁₈-alkylcarbonyl, C₂-C₁₉-alkenylcarbonyl, C₃-C₁₉-alkynylcarbonyl, C₆-C₂₀-aryl, C₆-C₁₄-aryl-C₁-C₈-alkyl, or a radical of the formula III 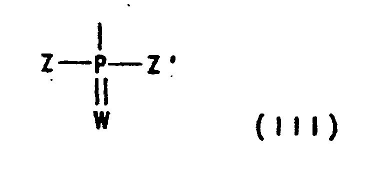
R² is hydrogen, hydroxyl, C₁-C₁₈-alkoxy, halogen, azido or NH₂;
D is hydroxyl, O-PO₃²⁻;
B is a base customary in nucleotide chemistry, for example natural bases or unnatural bases;
n is an integer from 7 to 25;
n' is an integer from zero to 50;
m' in formula I is an integer from zero to 5;
A is oxy, thioxy or methylene;
W is oxo, thioxo or selenoxo;
V is oxy or sulfanediyl;
T is oxy, sulfanediyl or imino;
Y is oxy, sulfanediyl, imino or methylene;
X is hydroxyl or mercapto;
U is hydroxyl, mercapto, BH₃, SeH, C₁-C₁₈-alkoxy, C₁-C₁₈-alkyl, C₆-C₂₀-aryl, C₆-C₁₄-aryl-C₁-C₈-alkyl, NHR³, NR³R⁴ or a radical of the formula IV
(OCH₂CH₂)ₚO(CH₂)_{q}CH₂R⁵ (IV)
in which
R³ is C₁-C₁₈-alkyl, C₆-C₂₀-aryl, C₆-C₁₄-aryl-C₁-C₈-alkyl, -(CH₂)_{c}-[NH(CH₂)_{c}]_{d}-NR⁶R⁶, in which c is an integer from 2 to 6 and d is an integer from zero to 6, and R⁶ is, independently of one another, hydrogen, C₁-C₆-alkyl or C₁-C₄-alkoxy-C₁-C₆-alkyl;
R⁴ is C₁-C₁₈-alkyl, C₆-C₂₀-aryl or C₆-C₁₀-aryl-C₁-C₈-alkyl, or, in the case of NR³R⁴, together with R³ and the nitrogen atom carrying them is a 5-6-membered heterocyclic ring which can additionally contain another hetero atom from the series consisting of 0, S and N;
P is an integer from 1 to 100;
q is an integer from zero to 22;
R⁵ is hydrogen or a functional group selected from hydroxyl, amino, NHR⁷, COOH, CONH₂, COOR⁸ or halogen, in which R⁷ is C₁-C₆-alkyl and R⁸ is C₁-C₄-alkyl;
Z, Z' are, independently of one another, hydroxyl, mercapto, SeH, C₁-C₂₂-alkoxy, -O- (CH₂)_{b}-NR⁷R⁸, in which b is an integer from 1 to 6, and R⁷ is C₁-C₆-alkyl and R⁸ is C₁-C₄-alkyl, or R⁷ and R⁸ form, together with the nitrogen atom carrying them, a 3-6-membered ring; C₁-C₁₈-alkyl, preferably C₁-C₈-alkyl, C₆-C₂₀-aryl, C₆-C₁₄-aryl-C₁-C₈-alkyl, preferably C₆-C₁₀-aryl-C₁-C₄-alkyl, C₆-C₁₄-aryl-C₁-C₈-alkoxy, preferably C₆-C₁₀-aryl-C₁-C₄-alkoxy, where aryl also means heteroaryl and aryl is optionally substituted by 1, 2 or 3 identical or different radicals from the series consisting of carboxyl, amino, nitro, C₁-C₄-alkylamino, C₁-C₆-alkoxy, hydroxyl, halogen and cyano, or C₁-C₁₈-alkylmercapto, NHR³, NR³R⁴, in which R³ and R⁴ are as defined above, or a group
a) which favors intracellular uptake, namely lipophilic radicals such as -O-(CH₂)ₓ-CH₃, in which x is an integer from 6 to 18, -O-(CH₂)ₑ-CH=CH-(CH₂)_{f}-CH₃, in which e and f independently of one another are an integer from 6 to 12, -0-(CH₂CH₂O)₄-(CH₂)₉-CH₃, -O- (CH₂CH₂O)₈-(CH₂)₁₃-CH₃ and -O-(CH₂CH₂O)₇-(CH₂)₁₅-CH₃, steroid residues such as cholesteryl and conjugates which utilize natural carrier systems such as bile acid, folic acid, 2-(N-alkyl-N-alkoxy)aminoanthraquinone and conjugates of mannose and peptides of the corresponding receptors which lead to the receptor-mediated endocytosis of the oligonucleotides, such as EGF (epidermal growth factor), bradykinin and PDGF (platelet derived growth factor), or
b) acts as labelling of a DNA probe, namely fluorescent groups, for example dansyl-(=N-dimethyl-1-aminonaphthyl-5-sulfonyl), fluorescein or coumarin derivatives or chemiluminescent groups, for example acridine derivatives, the digoxigenin system, the biotin group or linker arms having functional groups which permit subsequent derivatization with detectable reporter groups, for example an aminoalkyl linker which is reacted with an acridinium active ester to form a chemiluminescence probe, or
c) on hybridization of the oligonucleotide analog onto the target nucleic acid, interacts with the latter by binding, crosslinking or cleavage, namely acridine, psoralen, phenanthroline, naphthoquinone, daunomycin or chlorethylaminoaryl conjugates, or
d) a nucleoside or oligonucleotide linked via the 5' or 3' end; and
the curved parenthesis indicates that R² and the adjacent phosphoryl radical can be located in the 2' and 3' positions or else conversely in the 3' and 2' positions, it being possible for each nucleotide to be in its D or L configuration and for the base B to be located in the α or β position.

2. A compound of the formula I as claimed in claim 1, and the physiologically tolerated salts thereof, wherein the base B is located in the β position, the nucleotides are in the D configuration, and R² is located in position 2'.

3. A compound of the formula I as claimed in claim 1 or 2, and the physiologically tolerated salts thereof, wherein
a is a number from zero to 10;
b is a number from zero to 10;
R¹ is hydrogen or a radical of the formula III 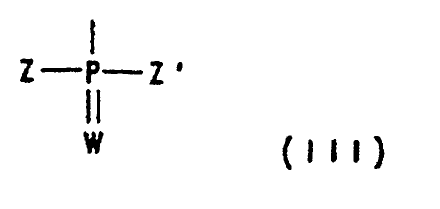
R² is hydrogen or hydroxyl;
n' is an integer from zero to 30;
A is oxy;
W is oxo or thioxo;
U is hydroxyl, mercapto, C₁-C₆-alkoxy, C₁-C₆-alkyl, NR³R⁴ or NHR³, in which
R³ is C₁-C₁₈-alkyl; and
R⁴ is C₁-C₈-alkyl, C₆-C₂₀-aryl or C₆-C₁₀-aryl-C₁-C₈-alkyl, or, in the case of NR³R⁴, together with R³ and the nitrogen atom carrying them is a 5-6-membered heterocyclic ring which can additionally contain another hetero atom from the series consisting of O, S, N.

4. A compound of the formula I as claimed in claims 1 to 3, and the physiologically tolerated salts thereof, wherein
a is a number from zero to 4;
b is a number from zero;
R¹ is hydrogen;
R² is hydrogen;
D is hydroxyl;
B is adenine, cytosine, guanine, uracil, thymine, 5-propinuracil or 5-propincytosine;
n' is an integer from zero to 25;
m is zero;
m' in formula I is an integer from zero or 1;
T is oxy;
Y is oxy; and
U is hydroxyl or C₁-C₆-alkyl.

5. The use of compounds of the formula I as claimed in claims 1 to 4 as probe for detecting nucleic acids in vitro.

6. A pharmaceutical composition comprising a compound of the formula I as claimed in claims 1 to 4, where appropriate together with physiologically tolerated ancillary substances and/or vehicles and/or together with other known active substances.

## Revendications

1. Composés de formule I 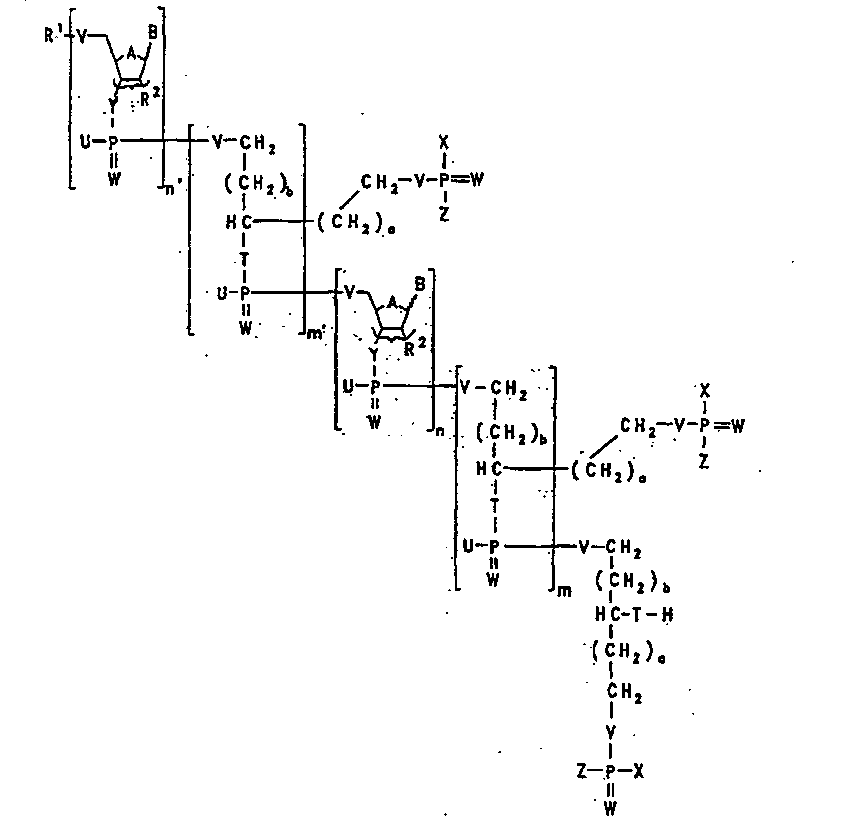 ainsi que leurs sels physiologiquement acceptables, où a signifie un nombre de 0 à 20;
b signifie un nombre de 0 à 20;
R¹ signifie hydrogène, alkyle en C₁ à C₁₈, alcényle en C₂ à C₁₈, alcynyle en C₃ à C₁₈, (alkyle en C₁ à C₁₈) carbonyle, (alcényle en C₂ à C₁₉) carbonyle, (alcynyle en C₃ à C₁₉) carbonyle, aryle en C₆ à C₂₀, (aryle en C₆ à C₁₄) (alkyle en C₁ à C₈) ou un radical de formule III 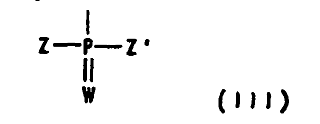 R² signifie hydrogène, hydroxy, alcoxy en C₁ à C₁₈, halogène, azido ou NH₂;
D signifie hydroxy, O-PO₃²⁻ ;
B représente une base usuelle pour la chimie des nucléotides, par exemple des bases naturelles ou des bases non naturelles ;
n signifie un nombre entier de 7 à 25 ;
n' signifie un nombre entier de 0 à 50 ;
m' dans la formule I signifie un nombre entier de 0 à 5 ;
A représente oxy, thioxy ou méthylène ;
W représente oxo, thioxo ou sélénoxy ;
V signifie oxy ou sulfanediyle ;
T signifie oxy, sulfanediyle ou imino ;
Y signifie oxy, sulfanediyle, imino ou méthylène ;
X signifie hydroxy ou mercapto ;
U signifie hydroxy, mercapto, BH₃, SeH, alcoxy en C₁ à C₁₈, alkyle en C₁ à C₁₈, aryle en C₆ à C₂₀, (aryle en C₆ à C₁₄) (alkyle en C₁ à C₈), NHR³, NR³R⁴ ou un radical de formule IV
(OCH₂CH₂)ₚO(CH₂)_{q}CH₂R⁵ (IV)
dans laquelle
R³ signifie alkyle en C₁ à C₁₈, aryle en C₆ à C₂₀, (aryle en C₆ à C₁₄) (alkyle en C₁ à C₈) -(CH₂)_{c}-[NH(CH₂)_{c}]_{d}-NR⁶R⁶, où c représente un nombre entier de 2 à 6 et d un nombre entier de 0 à 6, et R⁶ représente, indépendamment l'un de l'autre, alkyle en C₁ à C₆ ou (alcoxy en C₁ à C₄) (alkyle en C₁ à C₆) ;
R⁴ signifie alkyle en C₁ à C₁₈, aryle en C₆ à C₂₀ ou (aryle en C₆ à C₁₀) (alkyle en C₁ à C₈) ou, dans le cas de NR³R⁴, avec R³ et l'atome d'azote qui les porte un hétérocycle de 5-6 chaînons qui peut en outre contenir un autre hétéroatome de la série O, S, N ;
p représente un nombre entier de 1 à 100 ;
q représente un nombre entier de 0 à 22 ;
R⁵ signifie hydrogène ou un groupe fonctionnel choisi parmi hydroxy, amino, NHR⁷, COOH, CONH₂, COOR⁸ ou halogène, où R⁷ signifie alkyle en C₁ à C₆ et R⁸ représente alkyle en C₁ à C₄ ;
Z, Z' signifient, indépendamment l'un de l'autre, hydroxy, mercapto, SeH, alcoxy en C₁ à C₂₂, -O-(CH₂)_{b}-NR⁷R⁸, où b représente un nombre entier de 1 à 6, et R⁷ représente alkyle en C₁ à C₆ et R⁸ représente alkyle en C₁ à C₄ ou R⁷ et R⁸ forment ensemble, avec l'atome d'azote qui les porte, un cycle de 3-6 chaînons ; alkyle en C₁ à C₁₈, de préférence alkyle en C₁ à C₈, aryle en C₆ à C₂₀, (aryle en C₆ à C₁₄) (alkyle en C₁ à C₈), de préférence (aryle en C₆ à C₁₀) (alkyle en C₁ à C₄), (aryle en C₆ à C₁₄) (alcoxy en C₁ à C₈), de préférence (aryle en C₆ à C₁₀) (alcoxy en C₁ à C₄), aryle signifiant également hétéroaryle et aryle étant le cas échéant substitué par 1, 2 ou 3 radicaux identiques ou différents de la série carboxy, amino, nitro, (alkyle en C₁ à C₄) amino, alcoxy en C₁ à C₆, hydroxy, halogène et cyano, (alkyle en C₁ à C₁₈)mercapto, NHR³, NR³R₄, dans laquelle R³ et R⁴ sont tels que définis ci-dessus ou un groupe qui
a) favorise l'absorption intracellulaire, notamment les radicaux lipophiles tels que -O-(CH₂)ₓ-CH₃, où x signifie un nombre entier de 6 à 18, -O-(CH₂)ₑ-CH=CH-(CH₂)_{f}-CH₃, où e et f signifient, indépendamment l'un de l'autre, un nombre entier de 6 à 12, -O-(CH₂CH₂O)₄-(CH₂)₉-CH₃, -O- (CH₂CH₂O)₈-(CH₂)₁₃-CH₃ et -O-(CH₂CH₂O)₇-(CH₂)₁₅-CH₃, les radicaux de stéroïdes tels qu'un radical cholestéryle et les produits conjugués qui exploitent les systèmes support naturels, tels que l'acide galénique, l'acide folique, la 2-(N-alkyl-N-alcoxy)-aminoanthraquinone et les produits conjugués du mannose et les peptides des récepteurs correspondants, qui conduisent à l'endocytose, provoquée par les récepteurs, des oligonucléotides, tels que l'EGF (Epidermal Growth Factor), la bradykinine et le PDGF (Platelet Derived Growth Factor) ou
b) sert de marquage d'une sonde d'ADN, notamment les groupes fluorescents, par exemple les dérivés de dansyle (=N-diméthyl-1-aminonaphtyl-5-sulfonyl-), de fluorescéine ou de coumarine ou les groupes chimioluminescents, par exemple les dérivés d'acridine, le système digoxygénine, le groupe biotine ou les bras de liaison avec des groupes fonctionnels qui permettent une dérivation ultérieure avec des groupes rapporteurs détectables, par exemple une liaison aminoalkyle qui est transformée avec un ester actif d'acridinium en sonde de chimio-luminescence ou
c) s'agrippe lors de l'hybridation de l'analogue d'oligonucléotide à l'acide nucléique cible avec une liaison, une réticulation transversale ou une dissociation, notamment les produits conjugués d'acridine, de psoralène, de phénanthroline, de naphtoquinone, de daunomycine ou de chloroéthylaminoaryle ou
d) indique un nucléoside ou un oligonucléotide lié via l'extrémité 5'- ou 3' et
l'accolade indique que R² et le radical phosphoryle adjacent peuvent se trouver en position 2' et 3' ou également inversement en position 3' et 2',
chaque nucléotide pouvant se trouver dans sa configuration D ou L et la base B pouvant se trouver en position α ou β.

2. Composés de formule I selon la revendication 1 ainsi que leurs sels physiologiquement acceptables, **caractérisés en ce que** la base B se trouve en position β, les nucléotides se trouvent dans la configuration D et R² se trouve en position 2'.

3. Composés de formule I selon la revendication 1 ou 2, ainsi que leurs sels physiologiquement acceptables, **caractérisés en ce que**
a signifie un nombre de 0 à 10 ;
b signifie un nombre de 0 à 10 ;
R¹ signifie hydrogène ou un radical de formule III ; 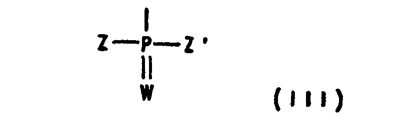 R² signifie hydrogène ou hydroxy ;
n' signifie un nombre entier de 0 à 30 ;
A représente oxy ;
W signifie oxo ou thioxo ;
U signifie hydroxy, mercapto, alcoxy en C₁ à C₆, alkyle en C₁ à C₆, NR³R⁴ ou NHR³, où
R³ signifie alkyle en C₁ à C₈ ; et
R⁴ signifie alkyle en C₁ à C₈, aryle en C₆ à C₂₀ ou (aryle en C₆ à C₁₀) (alkyle en C₁ à C₈), ou dans le cas de NR³R⁴, ensemble avec R³ et l'atome d'azote qui les porte, un hétérocycle de 5-6 chaînons, qui peut contenir en outre un autre hétéroatome de la série O, S, N.

4. Composés de formule I selon les revendications 1 à 3 ainsi que leurs sels physiologiquement acceptables, **caractérisés en ce que**
a signifie un nombre de 0 à 4 ;
b signifie un nombre de 0 ;
R¹ signifie hydrogène;
R² signifie hydrogène;
D signifie hydroxy;
B représente adénine, cytosine, guanine, uracile, thymine, 5-propynuracile ou 5-propynecytosine;
n' signifie un nombre entier de 0 à 25 ;
m signifie 0 ;
m' dans la formule I signifie un nombre entier de 0 ou 1 ;
T signifie oxy;
Y signifie oxy ; et
U signifie hydroxy ou alkyle en C₁ à C₆.

5. Utilisation des composés de formule I selon les revendications 1 à 4 comme sonde pour la détection d'acides nucléiques in vitro.

6. Composition pharmaceutique contenant un composé de formule I selon les revendications 1 à 4, le cas échéant avec des adjuvants et/ou des substances support physiologiquement acceptables et/ou avec d'autres substances actives connues.
